# EUROPEAN PATENT APPLICATION

(11) **EP 1 403 709 A2**
(43) Date of publication of application: **31.03.2004**
(21) Application number: 03256125.0
(22) Date of filing: 29.09.2003
(51) Int. Cl.: G03F 7/031

(54) **Photoinitiator**

(30) Priority: 30.09.2002 US 414759 P
(71) Applicant: Shipley Co. L.L.C., Marlborough, MA 01752 (US)
(72) Inventor: Barr, Robert K., Shrewsburg Massachusetts 01545 (US); Anzures, Edgardo, Westborough Massachusetts 01581 (US); Lundy, Daniel E., Winchendon Massachusetts 01475 (US)
(74) Representative: Kent, Venetia Katherine

(57) **Abstract**

A compound having a photoinitiator component joined to a hydrophilic carrier component. The carrier component may contain sufficient hydrophilicity such that the entire compound is water-soluble or water-emulsifiable. The compounds may be employed in light sensitive formulations such as photoresists.

## Description

### Background of the Invention

The present invention is directed to an improved photoinitiator. More specifically, the present invention is directed to an improved photoinitiator having a photoinitiator component joined to a hydrophilic carrier component.

Photoinitiators are employed in numerous chemical formulations to make the formulations light sensitive. When the chemical formulations are exposed to actinic radiation the chemical components cross-link or polymerize such as in photoresist formulations. Photoresists are photosensitive films used for transfer of images to a substrate. A coating layer of a photoresist is formed on a substrate and the photoresist layer is then exposed through a photomask to a source of activating radiation. The photomask has areas that are opaque to activating radiation and other areas that are transparent to activating radiation. Exposure to activating radiation provides a photoinduced chemical transformation of the photoresist coating to thereby transfer the pattern of the photomask to the photoresist-coated substrate. Following exposure, the photoresist is developed to provide a relief image that permits selective processing of a substrate.

A photoresist can be either positive-acting or negative-acting. For most negative-acting photoresists, those coating layer portions that are exposed to activating radiation polymerize or cross-link in a reaction between a photoactive compound and polymerizable agents of the photoresist composition. Consequently, the exposed coating portions are rendered less soluble in a developer solution than unexposed portions. For positive-acting photoresists, exposed portions are rendered more soluble in a developer solution while areas not exposed remain comparatively less developer soluble.

In addition to a photoinitiator or photoactive agent, photoresist compositions include at least a resin binder component, and a monomer or oligomer component. A wide variety of polymeric or resin binders may be used in photoresists. Such polymeric binders may include, as polymerized components, one or more acid functional monomers such as acrylic acid or methacrylic acid. Photoresists may be employed in a number of industries. Such industries include, but are not limited to, the electronics industry such as in the manufacture of printed wiring boards, photomasks, planographic printing plates and semiconductors, the manufacture of color filters for use in color liquid crystal display devices, color image pick-up elements, and the like. For example, U.S. Patent No. 5,952,153 (Lundy et al.) discloses photoimageable compositions containing polymeric binders having sufficient acid functionality to render the photoimageable composition developable in alkaline aqueous solution. U.S. Patent No. 4,537,855 (Ide) discloses polycarboxylic acids used to form polymerizable ester derivatives with ethylenically unsaturated compounds. Such polymerizable ester derivatives are used to form the polymeric binders for photoimageable compositions.

Monomers useful in photoresist compositions are any which are cross-linkable. Such monomers cross-link to form a polymerized network having a very large, i.e. infinite, molecular weight. The polymeric binders do not participate in such cross-linking. Rather, the monomers form a polymerized network around the polymeric binders. Polymeric binders may contain pendant groups, such as carboxylic acids that react with the developer to increase the water solubility of the binder. Thus, in the unexposed portion, the acid functional polymer is salted in the alkaline solution, while in the exposed area (protected by the cross-linked monomers), the polymer is not affected. During stripping, the polymerized network (of cross-linking monomers) is attacked or degraded by the stripper allowing it to be removed, whereas the polymeric binder remains relatively unaffected by such strippers.

Photoresists also may be either liquid or dry film. Liquid photoresists are dispensed on a substrate and then cured. Dry film photoresists may be laminated to a substrate. Such dry film photoresists are particularly suitable for use in printed wiring board manufacture. During processing of both negative-working and positive-working photoresists in the manufacture of printed wiring boards, photoresist that is not required in subsequent processing steps is removed with developer and stripper solutions. Developer and stripper solutions are usually contained in an immersion or spray apparatus. Often the uncured photoresist deposits organic scum and residue that adhere to equipment parts. If such scum and residue is not removed, the scum and residue may contaminate printed wiring boards, thus resulting in defective boards. Much of the organic scum and residue is caused by α,β-ethylenically unsaturated monomers and oligomers such as (meth)acrylate-based compounds, and photoactive agents having numerous aromatic groups in their structures. Examples of such photoactive agents that may form part of the scum and residue include, but are not limited to, imidazole dimers, benzophenones, acetophenones, anthraquinones, naphthaquinones, triazine-based compounds and the like. A number of these photoactive agents, such as the imidazole dimers, are hydrophobic. Especially desirable imidazole dimers for use in photoresists are the hexaarylbiimidazole compounds. However, such compounds are notorious for contributing to scum and residue formation. The scum and residue that form from such compounds on articles or apparatus are not readily water-soluble or readily water-emulsifiable. As dissolved photoresist builds up in solution (developer loading), insoluble organic materials begin to form in the developing tank eventually forming a water insoluble residue or scum. Presence of anti-foam additives (added to developer solutions to minimize foaming) greatly increases the tendency for residue and scum to form. As the level of scum builds, chances increase for a redeposit of the water insoluble residues onto the developed circuit board. Such redeposited residues cause a retardation of etching solution (etching chemistries have difficulty penetrating organic residues). Where etch is retarded, circuit shorts form causing a defective circuit board. In addition to increasing the potential for defective circuit boards, the residue also makes cleaning equipment difficult, thus increasing maintenance time and cost.

In addition to the problem of built-up residue and scum formation from primary photoresists, there also is a residue and scum build-up problem from secondary photoresists. Such secondary photoresists may be employed in soldermasks. Residue and scum are deposited on a substrate as a result of component separation in the soldermask. Such component separation may be exacerbated when an improperly balanced soldermask developer solution, i.e., improper developing conditions and/or soldermask developer solution chemistry, contact the soldermask. Built-up residue and scum from secondary photoresists may appear as a bright green coating on a substrate such as a developer apparatus.

Cleaners are available for removing residue and scum. Such conventional cleaners include as active ingredients strong bases such as sodium hydroxide, and chelating agents such as ethylene diamine tetraacetate (EDTA). Surfactants, solvents and emulsifying agents also may be included in the cleaners. Such cleaners are primarly used because of the low cost of their ingredients. However, workers in the field using such cleaners have discovered that the residue problem often is made worse. Often the equipment has to be manually cleaned to remove the residue from the uncured photoresist as well as residue from the cleaners. Such manual cleaning is both a labor and time intensive operation that can cause a significant loss of production time. Further, such cleaners are not effective enough for removing residue from many new generation photoresists that have numerous hydrophobic aromatic components.

U.S. 5,922,522 to Barr et al., U.S. 6,063,550 to Lundy et al., and U.S. 6,248,506 B1 to Lundy et al. disclose surfactant and surfactant mixtures included in developer solutions that prevent or inhibit the formation of residues and scum on circuit boards and circuit board manufacturing equipment. Such surfactants are composed of a hydrophobic group, an alkoxylated hydrophilic group and a nonionic or anionic capping group. Examples of suitable hydrophobic groups include nonylphenol, octylphenol and tristyrylphenol. Examples of suitable alkoxylated hydrophilic groups include ethylene oxide, propylene oxide and ethylene oxide/propylene oxide groups. Examples of suitable capping groups include hydroxyl, carboxyl, sulfonyl, or phosphonyl. Such residue and scum reducing compounds are included in developer solutions in amounts of from 0.05% to 1.0% by weight of the developer solution. Although the developer solutions disclosed in U.S. 5,922,522, U.S. 6,063,550 and U.S. 6,248,506 B1 provide an effective means of reducing the amount of build-up of residue and scum on substrates containing photoresist, there is still a need for a method of further preventing or reducing scum and residue build-up.

U.S. 6,180,319 B1 to McKeever discloses photoresist compositions having a photoinitiator of a hexarrylbiimidazole (HABI) compound having a hydrophilic group. Such hydrophilic groups are alkoxy groups, dialkylamino groups, carboxylic acid groups and their alkyl esters, amides and salts, or combinations of the foregoing groups. Alkoxy substituents are preferred with the methoxy the most preferred. The patent alleges that such groups attached to a HABI molecule employed in a photoresist help reduce the accumulation of sludge in recycled developer solutions and stripping solutions used in lithographic procedures.

Although there are compositions available for cleaning or preventing scum and residue formation deposited by photoresists, there is still a need for an improved photoinitiator that eliminates or at least reduces scum and residue formation.

### Summary of the Invention

The present invention is directed to a hydrophilic photoinitiator containing compound having a photoinitiator compound or component joined to a hydrophilic carrier compound or component.

Advantageously, the hydrophilic carrier component for the photoinitiator component is water-soluble or water-emulsifiable such that the photoinitiator component is water-soluble or water-emulsifiable, thus the entire photoinitiator containing compound is water-soluble or water-emsulifiable. The water-solubility or water-emulsifiability of the photinitiator containing compound eliminates or at least reduces accumulation of scum and residue in developer and stripper solutions as well as scum and residue deposits on apparatus and printed wiring boards. Many photoinitiator compounds are hydrophobic and contribute to the formation of scum and residue in lithographic procedures. By joining the hydrophobic photoinitiator compounds to a water-soluble or water-emulsifiable carrier, the photoinitiator components are rendered water-soluble or at least water-emulsifiable. Any photoinitiator with a reactive group that may undergo an addition or condensation reaction with a reactive group of the hydrophilic carrier compound to form a bond may be employed to practice the present invention. The elimination of or at least the reduction of scum and residue eliminates or reduces the need for cleaning compositions and the chance of additional contamination caused by such cleaning compositions. Also, lithographic processes are made more efficient since the manufacturing processes need not be shutdown for protracted cleaning procedures. Additionally, the number of defective printed wiring boards is reduced.

In another embodiment, the present invention is directed to a photoresist which includes the hydrophilic photoinitiator containing compound of the present invention. The photoinitiator component with a reactive group may be bonded to a hydrophilic carrier component such as a monomer, oligomer, polymer binder, an unreactive modifier such as a plasticizer, surfactant, or any other hydrophilic compound by means of an addition or condensation reaction with a reactive group of the carrier component, thus making the photoactive component water-soluble or water-emulsifiable. The water-solubility or water-emulsifiability of the hydrophilic photoinitiator containing compound prevents or at least reduces scum and residue formation from uncured photoresist in contrast to conventional photoresists in developer solutions, stripper solutions, or apparatus and on printed wiring boards. Another advantage of the photoresist is that additional photoinitiators need not be added to the photoresist formulation, thus avoiding leaching of photoinitiators from the uncured photoresist causing scum and residue formation. In addition to the water-soluble or water-emulsifiable photoinitiator containing compounds, the photoresists of the present invention also may have conventional additives.

Another embodiment of the present invention provides a method for forming a pattern on a substrate which includes the steps of: a) disposing on a substrate a photoresist composition which includes a hydrophilic photoinitiator containing compound; b) imaging the photoresist; and c) developing the photoresist.

A primary objective of the present invention is to provide a hydrophilic photoinitiator containing compound which prevents or at least reduces scum and residue formation.

Another objective of the present invention is to provide an improved photoresist which includes a hydrophilic photoinitiator containing compound which reduces or eliminates scum and residue formation.

A further objective of the present invention is to provide a method for forming an image on a substrate using a photoresist with a hydrophilic photoinitiator containing compound which prevents or reduces scum and residue formation.

Additional objectives and advantages of the present invention may be discernable to a person of skill in the art after reading the description of the invention and the appended claims.

### Detailed Description of the Invention

The present invention is directed to a hydrophilic compound having a photoactive component or photoinitiator component joined to a hydrophilic carrier component. The carrier component of the hydrophilic photoinitiator containing compound is a sufficiently water-soluble or water-emulsifiable, i.e., hydrophilic, compound such that the photoinitiator compound is water-soluble or water-emulsifiable. The water-solubility or water-emulsifiability of the photoinitiator containing compound eliminates or at least reduces accumulation of scum and residue in developer and stripper solutions as well as scum and residue deposits on apparatus and printed wiring boards from uncured photoresists in contrast to photoinitiators not joined to a hydrophilic carrier compound. Photoactive agents that may be joined to a hydrophilic carrier component are photoinitiators which have reactive groups that react with reactive groups of the carrier by an addition reaction or condensation reaction. Examples of such addition reactions include nucleophilic, electrophilic and free radical addition reactions. When the photoinitiator does not have a reactive group to react with a reactive group of the carrier, a suitable reactive group may be attached to the photoinitiator by any suitable addition or substitution reaction know in the art.

Carrier components of the present invention are any suitable compound which may be joined to a photoinitiator and make the combined components of the photoinitiator containing compound water-soluble or water-emulsifiable, or capable of polymerizing with water-soluble or water-emulsifiable monomers or oligomers to form a water-soluble or water-emulsifiable, i.e., hydrophilic, photointiator containing compound. Examples of suitable carrier components include, but are not limited to, acid functional monomers, non-acid functional monomers, urethane oligomers, polymers, unreactive modifiers such as surfactants, complex surfactants, or plasticizers. Preferably, monomers, oligomers and polymers have sufficient acid functionality, such as carboxylic acid functionality, to form water-soluble salts, such as sodium or potassium salts, in developer or stripper solutions. Such developer and stripper solutions typically are basic aqueous solutions. Hydroxyl groups on the carrier component also add to the hydrophilicity. Oligomers with alkoxylations, such as ethoxylations and propoxylations also are suitable to add hydrophilicity to a carrier component. Examples of suitable urethane oligomers which may be employed to practice the present invention are described in U.S. 6,045,973, U.S. 6,166,245. U.S. 6,207,347 B1, U.S. 6,268,111 B1, U.S. 6,319,653, U.S. 6,322,951 B1 and U.S. 6,329,123 B1, the entire disclosures of which are hereby incorporated herein in their entireties by reference. Such monomers and oligomers may be employed in formulations where photosensitive compounds are desired. Additionally, the monomers and oligomers with photoinitiators may be polymerized to form polymer binders for photosensitive formulations. The term "polymer" within the scope of the present invention also encompasses copolymer.

Examples of suitable acid functional monomers are acrylic acid, methacrylic acid, maleic acid, fumaric acid, citraconic acid, 2-acrylamido-2-methylpropanesulfonic acid, 2-hydroxyethyl acrylolyl phosphate, 2-hydroxypropyl acrylol phosphate, 2-hydroxy-alpha-acryloyl phosphate, etc.

Non-acid functional monomers include, but are not limited to, esters of acrylic acid and methacrylic acid, for example, methyl acrylate, 2-ethyl hexyl acrylate, n-butyl acrylate, n-hexyl acrylate, methyl methacrylate, hydroxy ethyl acrylate, butyl methacrylate, octyl acrylate, 2-ethoxy ethyl methacrylate, t-butyl acrylate, 1,5-pentanediol diacrylate, N,N-diethylaminoethyl acrylate, ethylene glycol diacrylate, 1,3-propanediol diacrylate, decamethylene glycol diacrylate, decamethylene glycol dimethacrylate, 1,4-cyclohexanediol diacrylate, 2,2-dimethylol propane diacrylate, glycerol diacrylate, tripropylene glycol diacrylate, glycerol triacrylate, 2,2-di(p-hydroxyphenyl)-propane dimethacrylate, triethylene glycol diacrylate, polyoxyethyl-2-2-di(p-hydroxyphenyl)-propane dimethacrylate, triethylene glycol dimethacrylate, polyoxypropyltrimethylol propane triacrylate, ethylene glycol dimethacrylate, butylene glycol dimethacrylate, 1,3-propanediol dimethacrylate, butylene glycol dimethacrylate, 1,3-propanediol dimethacrylate, 1,2,4-butanetriol trimethacrylate, 2,2,4-trimethyl-1,3-pentanediol dimethacrylate, pentaerythritol trimethacrylate, 1-phenyl ethylene-1,2-dimethacrylate, pentaerythritol tetramethacrylate, trimethylol propane trimethacrylate, 1,5-pentanediol dimethacrylate, and 1,4-benzenediol dimethacrylate; styrene and substituted styrene, such as 2-methyl styrene and vinyl toluene and vinyl esters, such as vinyl acrylate and vinyl methacrylate to provide the desired acid number. The acid functional monomers may be copolymerized with the non-acid functional monomers to provide water-soluble or water-emulsifiable oligomers when the non-acid functional monomer is not sufficiently water-soluble or water-emulsifiable.

Specific examples of urethane oligomers include, but are not limited to, the following general formulas I and II.

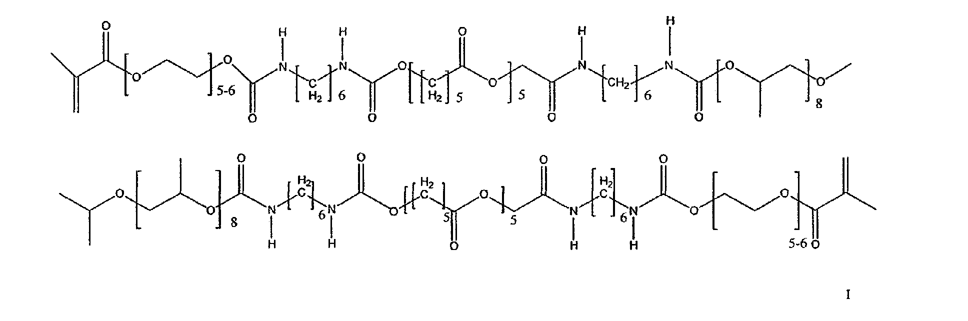

Urethane oligomers of formula I (single structure) may be prepared by any suitable method known in the art. An example of one method for forming the urethane oligomer of formula I involves reacting one mole of polyethylene glycol 300 monomethyacrylate with four moles of caprolactone in a chain opening extension reaction. An acid catalyst may be employed, and the reaction may be run at room temperature, i.e., 20° to 22°C. The product of the reaction is a polycaprolactone extension of polyethylene glycol 300 (block copolymer).

In a separate container, one mole of polypropylene glycol (molecular weight = about 1000) is reacted with two moles of 1,6-hexamethylene diisocyanate using known isocyanate chemical reaction conditions and methods. The products of the reaction is a urethane prepolymer terminated with an isocyanate.

To form the final end products, which is the urethane of formula I, two moles of the polycaprolactone extension of polyethylene glycol 300 is reacted with one mole of the isocyanate terminated urethane prepolymer. The reaction is a classic urethane condensation reaction well known in the art. The resulting product is the dimethyacrylate oligomer of formula I.

T-I'-(-P'-I'-)_{q}-T II

where I' is an aliphatic, cycloaliphatic, or aromatic isocyanate radical having an isocyanate functionality of 2 or greater, preferably an isocyanate derived radical of the formula: 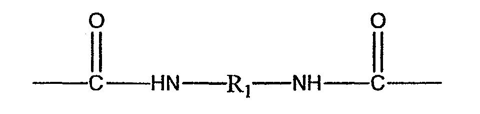 where R₁ is an aliphatic, cycloaliphatic, or aromatic hydrocarbon radical, with hexamethylene, cyclohexylene, and phenylene preferred, T is a radical of the formula: 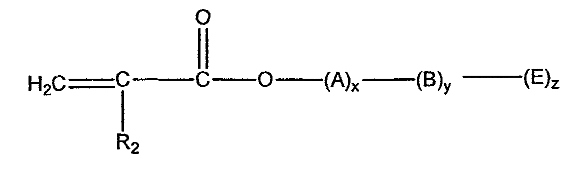 where R₂ is hydrogen or methyl, A, B and E are in the order given or in any order, preferably in the order given, A may be an alkylene oxide derived radical of the formula:
―[― (CH₂)ₙ ―O―]―, where n is an integer from 1 to 20, preferably 2 to 4, and x is an integer from 1 to 40, B may be an alkylene oxide derived radical of the formula: ―[― (CH₂)ₙ₁ ―O― ]―, where n1 is an integer from 1 to 20, preferably 2 to 4, and y is an integer from 0 to 40, with the alkylene oxide derived radicals, preferably from 4 to 12, and E is derived from lactone and is an open ring lactone radical of the formula: 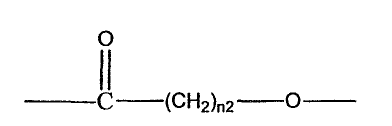 where n2 is an integer from 1 to 20, preferably 3 to 5, and z is an integer from 1 to 40, preferably 3 to 8 and q is 0 or an integer from 1 to 10.

In the above, if q is 1 or more, P' is: ―[―O―G ―]― where G is of the formula:
(A)_{*s*1}―(B)_{*s*2}―(E)_{*s*3}―(D)_{*f*}―(w)_{*k*}―(J)_{*u*}―(B)_{*s*4}―(A)_{*s*5}―, where A, B, E and R₁ are defined above and f, k, and u are 0 or 1, D is a radical of the formula: 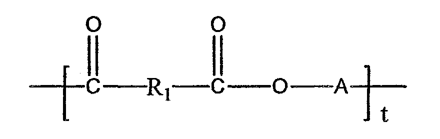 where t is an integer from 1 to 40, W is a radical of the formula: 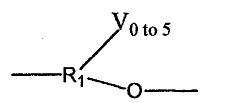 where V is an acidic group selected from ―COOH, ―SO₃ OH, and PO₃HR₃, R₃ is hydrogen or a C₁₋₁₈ alkyl radical, J is an ester functional alkyl radical of the formula: 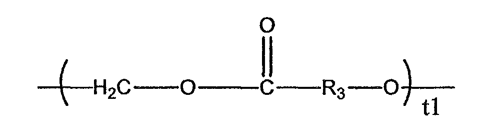 where t1 is an integer from 1 to 6, and, with the proviso that if f+k+u=0, then Σ_{*S*}_{1...}_{*S*}₅ must be >1.

Urethane oligomers of formula II may be prepared by any suitable method. A preferred method of forming urethane oligomers defined in the above formula III where q=0 is to initially block copolymerize one or more lactone derived radicals and two or more alkylene oxide derived radicals onto a (meth)acrylic acid backbone by a conventional addition polymerization procedure, to produce the (meth)acrylate-functional group of formula T which has a hydroxy-terminus opposite the (meth)acrylate functionality. The term "(meth)acrylate" refers to both methacrylate and acrylate.

While in the above described formula for T, (A), (B) if present, and (E) may theoretically be in any order, the preferred mode of oligomer synthesis dictates that (E) be at the terminus opposite the (meth)acrylate functionality. In the preferred synthetic route, (meth)acrylic acid is reacted with an alkylene oxide monomer to produce (A). If desired, further reaction is carried out with a different alkylene oxide monomer to produce (B). The resulting product is then reacted with a lactone monomer or mixture of lactone monomers to produce (E).

Alkylene oxide monomers used in forming the (A) and (B) alkylene oxide radicals may contain 1 to 20 carbon atoms, although short chain alkylene oxides of at least 2 up to 4 carbon atoms, such as ethylene oxide, propylene oxide, and butylene oxide, with ethylene oxide and propylene oxide being preferred. While (B), if present, is herein defined as is (A), (B) is formed from a different monomer from (A). For example (A) may be formed from ethylene oxide and (B) could be formed from propylene oxide, or (A) may be formed from a mixture of ethylene oxide and butylene oxide while (B) may be formed from a mixture of propylene oxide and butylenes oxide. The optional incorporation of (B) allows the oligomer to be tailored to particular applications. To provide sufficient chain length to the oligomer, (A) plus (B) must be formed from at least 2 alkylene oxide monomers total, preferably between 4 and 12 monomers. In addition to alkylene oxides, (A) and (B) may be derived from tetrahydrofuran or styrene oxide with one or more alkylene oxide.

Lactone derived component (E) of the (meth)acrylate functional oligomeric group defined in formula T is formed from 1 to 40 lactone monomer units, either a single lactone species or mixture of lactone species. The lactone species employed may have from 1 to 20 carbon atoms (not including the carbonyl carbon), although 3 to 5 carbon atom species are preferred. Epsilon-caprolactone is a preferred lactone for forming (E). Other suitable lactones include, but are not limited to, beta-butyrolactone, zeta-enantholactone, delta-valerolactone. Also, C₁-C₆ alkyl-substituted lactones, such as the alkyl delta-valerolactones, such as methyl-, ethyl-, hexyl-, dimethyl-, diethyl-, di-n-propyl-, di-n-hexyl-, di-iso-propyl-, trimethyl-, triethyl-, and tri-n-propyl-epsilon caprolactones, as well as C₁-C₆ alkoxy- and aromatic-substituted lactones may also be used.

The oligomer thus formed is hydroxy-terminated at the terminus opposite the (meth)acrylate functionality. Subsequently, the hydroxy-terminated (meth)acrylate oligomer is reacted with a polyfunctional isocyanate used in forming I' in the above formula by a conventional urethane addition polymerization procedure, to produce a (meth)acrylate-functional urethane oligomer component.

In the urethane reaction, the conditions are chosen so that the hydroxy terminal functionality of the (meth)acrylate functional oligomer reacts with all of the isocyanate functionalities present in I', to end-cap each of the isocyanate groups with T.

A preferred method for forming of urethane oligomers where q≥1 is to initially react an isocyanate with an alcohol by a conventional addition polymerization procedure, to form a polyisocyanate/polyol adduct represented by the formula I'― (P'―I')_{*q*}. Reaction conditions are chosen to form an isocyanate-terminated urethane oligomer to the virtual exclusion of alcohol-terminated polymeric materials. The optional incorporation of the (P'―I')_{*q*} group into the urethane oligomer of formula II allows the oligomer to be tailored to particular applications. Subsequently, the isocyanate-terminated adduct is reacted with the hydroxy-terminated (meth)acrylate oligomer T defined above. The process described above is disclosed in U.S. 6,322,951 B1, the entire disclosure of which is hereby incorporated herein its entirety by reference.

Examples of suitable alcohols include, but are not limited to, monomeric or polymeric diols, such as ethylene glycol, propylene glycol, 1,4-butanediol, 2-ethyl-1,6-hexanediol, 1,10-decanediol, 1,4-bis-hydroxymethylcyclohexane, diethylene glycol, triethylene glycol, polyethylene glycols having molecular weights (MW) from about 200 to 1,500, the reaction products of 4,4'-dihydroxydiphenyl ether, 4,4'-dihydroxydiphenyl sulfide, 4,4'-dihydroxydiphenyl methane, 4,4'-dihydroxydiphenylpropane or 4,4'-dihydroxydiphenylsulfone with 0 to 40 moles of alkylene oxide, polypropylene glycols, polytetrahydrofuran, polybutylene glycols, thioethylene glycol and dithiotriethylene glycol; polyester polyols, such as polycaprolactone, polybutyrolactone, polyethylene terephthalate, polypropylene adipate, polybutylene adipate, polyethylenebutylene sebacate, and other polyester polyols having molecular weights (Mw) in the range of about 250 to 3,000. Apart from the diols, monomer or polymeric compounds having 2 to 6 aliphatic hydroxy groups, such as glycerol, trimethylolpropane, pentaerythritol, dipentaerythritol, sorbitol, or polyalkoxylate derivatives of these; polymeric polyester polyols including the lactone polyesters; block copolymers of polyethers and polyesters having terminal hydroxy groups; and, caprolactone polyols and polysiloxane polyols, or the like. Other polyfunctional alcohols which contain acidic groups may also be used, including those described in U.S. Pat. No. 5,415,972, the entire disclosure of which is hereby incorporated herein its entirety by reference.

Through use of the above-described (meth)acrylate-functional urethane oligomer of formula II where the chain which links the (meth)acrylate functionality to the urethane group is extended with at least two alkylene oxide groups and at least one ring-opened lactone group, improved flexibility and tenting strength of the cross-linked system is achieved. The flexibility is achieved by incorporation of a long chain attached to the crosslinkable ethylenically unsaturated (meth)acrylate functionality. Coupled with improved flexibility, the urethane oligomer of formula II improves the adhesive properties of the resist to the copper clad surface of blank circuit board following lamination. Better adhesion enables the production of a fine line (less than 75 microns) resist sidewall that adheres better to the copper surface of the circuit board.

An improvement is seen in stripping and chemical resistance to processing solutions. Because the urethane oligomer of formula II produces better adhesion, stripping the resist from the copper surface would be expected to be more difficult. While not wishing to be bound by any particular theory, it is believed that by distancing the (meth)acrylate functionality from the urethane block with not only flexible alkylene oxide groups but also durable and high modulus ring-opened lactone groups, the ester links present in the ring-opened lactone portion provide sites for hydroxide attack during the stripping operation, thereby greatly shortening stripping time. While producing sites for stripping solution attack, the relatively hydrophobic chain extension also provides good chemical resistance to alkaline developing solution, acid plating baths and acid etching solutions.

Another oligomer suitable to practice the present invention may be an oligomer with isocyanate derived pendent functional groups. The functionalized oligomer has a main chain or backbone that is derived from ethylenically or acetylenically unsaturated polymerizable monomers, and at least one monomer employed to make the oligomer backbone has a group that is free to react with the isocyanate group of an isocyanate compound to join the isocyanate compound to the oligomer backbone to form a pendent functional group. At least one pendent functional group terminates in one or more α,β-ethylencially or acetylenically unsaturated groups. Isocyanates include monoisocyanates, diisocyanates, triisocyanates, and polyisocyanates. Such isocyanates include aliphatic, alicyclic, aromatic as well as heterocyclic isocyanate compounds.

The isocyanate derived oligomers may be prepared by a post polymerization functionalization process. In post polymerization functionalization, the main chain or backbone of the oligomer and the isocyanate derived functional pendent components are prepared separately. After the preparation of each of the separate components that make up the oligomer, they are then joined together in a separate reaction process to form the final functionalized oligomer product. The process is described below.

The main chain or backbone of the isocyanate functionalized oligomers may be derived from monomers which include, but are not limited to, acid functional monomers, base functional monomers, water soluble functional monomers or mixtures thereof. Examples of suitable ethylenically or acetylenically unsaturated monomers include, but are not limited to:
(meth)acrylic acid, (meth)acrylamides, alkyl (meth)acrylates, alkenyl (meth)acrylates, aromatic (meth)acrylates, vinyl aromatic monomers, nitrogen-containing compounds and their thio-analogs, substituted ethylene monomers, cyclic olefins, or substituted cyclic olefins. Preferred monomers include (meth)acrylic acid, alkyl (meth)acrylates and vinyl aromatic monomers. The backbone may be prepared by free radical polymerization or other suitable method.

After polymerization of monomers to form the oligomer backbone, the oligomer is reacted with free isocyanate groups of the isocyanate compounds. Free isocyanate, or unreacted isocyanate, i.e., -N=C=O, reacts with a hydroxyl group from the polymer backbone, or a hydroxyl group from a carboxyl group from the polymer backbone to form a R₄-NH-C(O)-P" linkage where P" is the oligomer backbone, and R₄ is an organic pendent group from the isocyanate compound which may terminate in one or more ethylenically or acetylenically unsaturated moieties. A moiety within the scope of the present invention means a component part of a compound. A free isocyanate that reacts with a primary or secondary amine moiety joined to the polymer backbone forms a R₄-NH-C(O)-NR₅-G'-P" linkage where R₅ includes, but is not limited to, hydrogen, a linear, branched or unsubstituted or substituted alkyl, or an unsubstituted or substituted aryl. Substituent groups R₅ include, but are not limited to, halogen, such as fluorine, bromine, chloride or iodine, hydroxyl, carboxyl, or a primary or secondary amine. A substituent group replaces a hydrogen on a carbon atom. G' is an organic moiety that joins the nitrogen to the polymer chain. G' includes, but is not limited to, an alkyl, or a substituted aryl where the nitrogen is joined to the aryl by an alkyl chain. The alkyl chain of G' may be a linear or branched (C₁-C₂₄) alkyl. A free isocyanate that reacts with a polyalkoxylated moiety from the polymer backbone forms a R₄-NH-C(O)-O(A₁O)_{x'}-C(O)-P" linkage where A₁ is a linear or branched (C₁-C₂₄)alkyl, and x' is an integer from 0 to 1,000, preferably from 1 to 200.

The isocyanate compound may be joined to the oligomer by any suitable method. For example, the oligomer backbone is mixed with an isocyanate compound at reaction temperatures below 80° C. Preferably, reaction temperatures are run at mild temperatures of from 20° C to 60° C. Mixing and heating are continued until the reaction is complete. Typically, the reaction continues for about 1 hour to less than 8 hours, preferably from about 4 to about 6 hours. Advantageously, the method is performed over short time periods, thus less energy is utilized in preparing the functionalized oligomer. Reactions that take place occur between a free isocyanate group on the isocyanate compound and a hydroxyl group, carboxyl group, or primary or secondary aminyl functional group attached to the oligomer main chain or backbone. One mole of free isocyanate reacts with one mole of a hydroxyl, carboxyl, or primary or secondary aminyl on the oligomer main chain. The reaction may be self quenching. There is believed to be no source of free radicals, or a source of cations or anions at the end of the reaction between the isocyanate group(s) and hydroxyl, carboxyl, or aminyl group(s) on the oligomer backbone. As a precaution water, alcohol, or other chemical speicies with a labile hydrogen, and a suitable catalyst, such as triethylamine, may be added at the end of the reaction to quench any free isocyanate. Also, a suitable polymerization inhibitor may optionally be added to prevent premature cross-linking of terminal ethylenically or acetylenically unsaturated moieties such as a (meth)acrylate moiety. Reaction completion may be determined by using standard analytical instruments well known in the art.

The methods of making a functional oligomer may be carried out in the presence of an inert dry solvent, for example, an ether such as diisopropyl ether, ethylene glycol dimethyl ether, diethylene glycol dimethyl ether, 1,4-dioxane, tetrahydrofuran or 1,2-dimethoxy propane; an ester such as butyrolactone, ethylene glycol carbonate or propylene glycol carbonate; an ether ester such as methoxyethyl acetate, ethoxyethyl acetate, 1-methoxypropyl-2-acetate, 2-methoxypropyl-1-acetate, 1-ethoxypropyl-2-acetate or 2-ethoxypropyl-1-acetate; ketones such as acetone or methyethyl ketone; nitriles such as acetonitrile, propionitrile or methoxypropionitrile; sulfones such as sulfolane, dimethylsulfone or diethylsulfone; and phosphoric acid esters such as trimethyl phosphate or triethyl phosphate. The processes also may be carried out without such solvents.

R₄ as described above is the isocyanate compound less the free isocyanate group that reacts with a functional group of the oligomer backbone, i.e., hydroxyl, carboxyl or aminyl group. Examples of such compounds include, but are not limited to, the following general formulas:

O=C=N-Z-NH-C(O)-O-Y-C(O)-CR₆=CH₂;

O=C=N-Z-N[-C(O)-NH-Z-N-C(O)-O-Y-O-C(O)-CR₆=CH₂]₂;

or 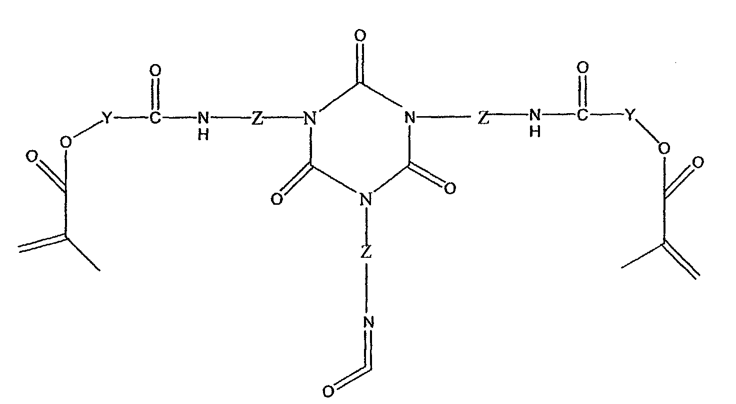 where Z includes, but is not limited to, alkyl, alkylene, cycloalkyl, aryl, heterocyclic alkyl, heteroaryl, a polymer such as a copolymer including a branched polymer or branched copolymer; Y includes, but is not limited to, alky, alkylene, cycloalkyl, aryl, heterocyclic alkyl, heteroaryl, -((CH₂)ᵣ-O-)ᵥ-(CH₂)_{w}-, or -((CH₂)ᵣ-C(O)-O-)ᵥ-(CH₂)_{w}-, where r, and w are integers of from 1 to 10, and v is an integer of from 0 to greater than 1,000, preferably from 1 to 200, most preferably from 5 to 10. R₆ is hydrogen or (C₁-C₄) alkyl. Preferably R₆ is hydrogen or methyl. Heteroatoms include, but are not limited to, oxygen, sulfur, and nitrogen. The alkyl, alkylene, cycloalkyl, aryl, heterocyclic alkyl, heteroaryl and polymers may be unsubstituted or substituted. Examples of suitable substitutent groups include, but are not limited to, carboxyl, hydroxyl, (C₁-C₄) alkyl, aminol such as a primary or secondary aminol, or hydroxy-aminol, or -CN.

Examples of suitable alkyl groups include, but are not limited to, linear or branched (C₁-C₂₀) alkyl. Examples of alkenyl, cycloakyl or aryl groups include, but are not limited to, linear or branched (C₂-C₂₀) alkenyl, (C₅-C₆) cycloalky such as an isophorone, and (C₅-C₆) aryl such as phenyl.

The isocyanate compounds with at least one free isocyanate group may be prepared by any suitable method known in the art. Diisocyanates or triisocyanates that may be employed are either known or may be prepared by analogy to known compounds. Examples of suitable diisocyanates and triisocyanates include, but are not limited to, ethylene diisocyanate, propylene diisocyanate, butylene-1,3-diisocyanate, 1,6-hexamethylene diisocyanate, 2,2,4-trimethyl-hexamethylene diisocyanate, 2,4-dimethyl-6-ethyloctamethylene diisocyanate, cyclohexylene diisocyanate, cyclopentylene diisocyanate, 1,4-diisocyanatomethyl-cyclohexane, 1,3-diisocyanatoethyl-cyclohexane, toluylene diisocyanate, 3,3,5-trimethyl-1-isocyanato-5-isocyanatomethyl-cyclohexane, 2-butene-1,4-diisocyanate, isophorone diisocyanate, 1,6-hexamethylene diisocyanate biuret, 1,6-hexamethylene diisocyanate trimer, isophorone diisocyanate trimer, bis phenol A dimethacrylate capped with 2-hydroxethylmethacrylate capped with 1,6-hexamethylene diisocyanate trimer, and the like. Many of the foregoing listed diisocyantes and triisocyantes as well as the biurets and trimers may be purchased from Lyondell (located at 122 Melanney St., Houston, TX) or Bayer (located at 100 Bayer Rd., Pittsburgh, PA 15025).

Isocyanates such as the diisocyanates and triisocyanates described above may then be reacted with a sufficient amount of one or more hydroxyl containing compounds such that at least one free isocyanate group is left to react with the main chain or backbone of the oligomer prepared as described above. The reaction mole ratio of hydroxyl group to isocyanate group is about 1:1. The process of addition of alcohols to isocyanates is well known in the art. Any suitable compound with at least one free hydroxyl group to react with an isocyanate group may be employed. An isocyanate compound also may be reacted with a second isocyanate compound having at least one free hydroxyl group. Hydroxyalkyl, hydroxyalkenyl, hydroxyaryl compounds and the like are examples of such compounds that may be employed. Hydroxyalkyl (meth)acrylates are one example of suitable compounds. Hydroxyethyl (meth)acrylate or hydroxypropyl (meth)acrylate (n or iso compounds) are examples of hydroxyl group-containing esters that are suitable. Other suitable hydroxyalkyl (meth)acrylates include, but are not limited to, 2-hydroxy-butyl (meth)acrylate, 4-hydroxy-butyl (meth)acrylate, 2-hydroxy-cyclohexyl (meth)acrylate, 2-hydroxyethylmethacrylate, and the like. Suitable polyethylene glycol mono (meth)acrylates also may be employed such as, but not limited to, diethylene glycol mono (meth)acrylate, triethylene glycol mono (meth)acrylate and the like. Hydroxyalicyclic (meth)acrylates, and hydroxyaromatic (meth)acrylates such as bis phenol A dimethacrylate also may be employed.

In addtion to the monomers, oligomers and polymers described above, examples of other suitable carrier components include, but are not limited to, plasticizers, surfactants, and complex surfactants. One example of a suitable plasticizer which may be employed to practice the present invention is a urethane plasticizer having formula III. 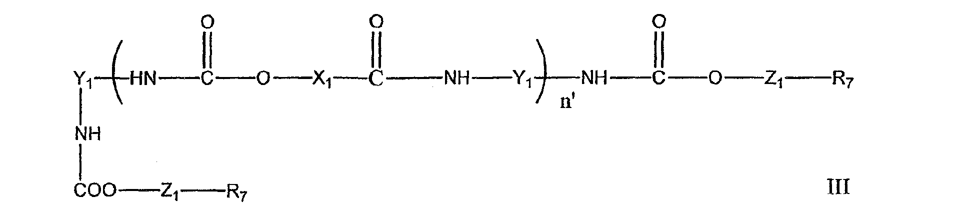 wherein
―X₁― is one of the following groups:

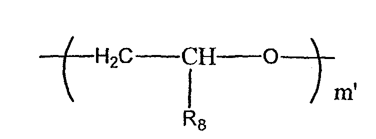

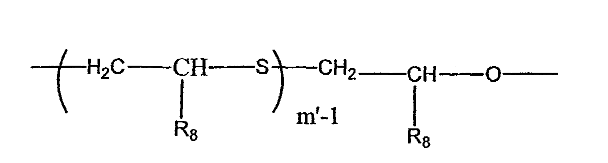

   ―C_{k'}H_{2k'}―O―

   and

   ―C_{r'}H_{2r'-2}―O―
Y₁ is a saturated apliphatic or cycloaliphatic group with 2 to 12 carbon atoms,
Z₁ is the group 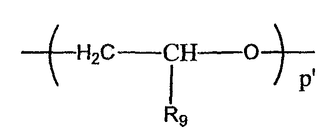
R₇ is R₁₀ or CONH―R₁₀,
R₈ and R₉ are hydrogen atoms or methyl groups,
R₁₀ is a saturated aliphatic group with 1 to 20 carbon atoms,
n' is zero or a whole number from 1 to 15,
m' is whole number from 2 to 4,
p' is zero or a whole number from 1 to 4,
k' is a whole number from 2 to 12,
r' is a whole number from 4 to 12, and
n' + p' is a whole number from 1 to 10, and wherein R₇=R₁₀ if p'=0, R₇ =CONH―R₁₀ if n'=0.

Because the urethane of formula III terminates in aliphatic or cycloaliphatic groups, a functional group such as a halogen may be added to at least one of the terminal groups in order for the urethane to react with a photoinitiator. The photoinitiator may have a hydroxyl group to react with the halogen and then form a bond with the urethane.

Examples of water-soluble or water-emulsifiable surfactants that may be employed to practice the present invention include alkoxylated emulsifiers and alkylglycosides or alkylpolyglycosides. A suitable alkoxylated emulsifier is one having the general formula:

R₁₁―O―(A'O)_{n''}―H IV

where A'O are alkylene oxide units selected from ethylene oxide units (CH₂―CH₂―O) and propylene oxide units (CH(CH₃)―CH₂―O) or (CH₂―CH(CH₃)―O) and mixtures of ethylene and propylene oxide units, either in the mixture of molecules, where R₁₁ is a hydrophobic group, typically a hydrocarbon group, n" is between 8 and 200, preferably between 15 and 40.

Preferably, R₁₁ is a tristyrylphenol.

Exemplary alkylglycoside surfactants suitable for use in the practice of this invention include those which may be represented by the formula: 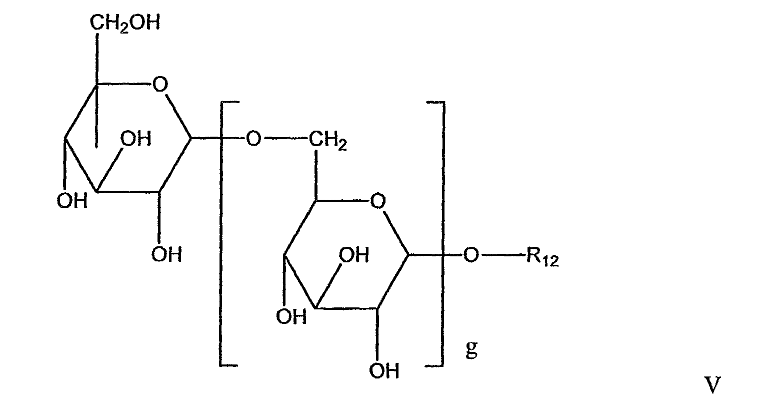 wherein:
R₁₂ is an alkyl group, preferably a linear alkyl chain, which comprises C₈ to C₁₆ alkyl groups;
g is an integer value from 0-3, inclusive.

Examples of such alkylpolyglycoside compounds according to this structure include: where R₁₂ is comprised substantially of C₈ to C₁₀ alkyl chains yielding an average value of about 9.1 alkyl carbons per molecule (Glucopon® 220 UP, Glucopon® 225 DK); where R₁₂ comprised of C₈, C₁₀, C₁₂, C₁₄ and C₁₆ alkyl chains yielding an average value of about 10.3 alkyl carbons per molecule (Glucopon® 425); where R₁₂ is comprised C₁₂, C₁₄ and C₁₆ alkyl chains yielding an average value of about 12.8 alkyl carbons per molecule (Glucopon® 600 UP, Glucopon® 625 CSU, and Glucopon® 625 FE, all of which are available from Henkel Corp., Ambler Pa.). Also useful as the alkylpolyglycoside compound is Triton® CG-110 (Union Carbide Corp.).

A particularly preferred alkylglycoside is GLUCOPON 325N which is described as being a C₉-C₁₁ alkyl polyglycoside, also commonly referred to a D-glucopyranoside (from Henkle Cop., Ambler Pa.).

The above specified carrier components are not limiting. Any suitable compound which is water-soluble or water-emulsifiable and that has a reactive group which may react with a photoinitiator to form a hydrophilic photoinitiator containing compound may be employed. Examples of such reactive groups include hydroxyl, carboxyl, aminyl, isocyanate, diisocyanate, epoxide, anhydride, amine, urea, or alkoxy. Other reactive groups are envisioned as long as a bond may be formed between the photoinitiator and the carrier component.

Photoinitiators may be joined to the oligomers described above directly, or indirectly by first joining the photoinitiator to another polyermizable carrier component then polymerizing with the oligomer to form a water-soluble or water-emulsifiable photoinitiator containing compound. Photoinitiators not joined to the carrier component are sufficiently hydrophobic to contribute to the scum and residue formation when used in photosensitive formulations, especially in photoresist formulations. Hydrophobic within the scope of the present invention means antagonistic to water such that the photoinitiator does not readily dissolve in water or is not emulsifiable with aqueous solutions. Water-soluble within the scope of the present invention means that a compound forms an aqueous solution of greater than 0.1% by weight. Preferably the compound forms an aqueous solution of greater than 1.0% by weight. Water-emulsifiable means that a compound may form an aqueous composition with dispersed particles in amounts of greater than 1.0% by weight, preferably greater than 1.0% by weight. Such photoactive components may deposit as a scum or residue in aqueous developer solutions, aqueous stripper solutions as well as on apparatus used in the manufacture of printed wiring boards as well as on the boards themselves. Scum and residue may be observed as a yellowish scum floating in aqueous developer or aqueous stripper solutions, or as a solid blue-green residue on apparatus and printed wiring boards. Such developer and stripper solutions are typically basic aqueous solutions. Accordingly, the hydrophobic photoinitiator containing compounds of the present invention are water-soluble or water-emulsifiable in basic aqueous solutions.

Hydrophobic photoinitiators may have a number of aromatic structures in their molecules which contribute to their hydrophobic nature. Examples of such photoinitiators include, but are not limited to, imidazole dimers, benzophenones, acetophenones, anthraquinones, naphthaquinones, or triazine-based compounds. Especially problematic photoinitiators are the imidazole dimers such as the hexaarylbiimidazoles also known as hexaarylbisimidazoles or abbreviated as HABI. Although such photoinitiators may be a major cause for the formation of scum and residue problems, the heaxaarylbiimidazoles are very useful in photosensitive formulations, especially in photoresists. Accordingly, an improvement in the water-solubility or water-emulsifiability of such photoinitiators is highly desirable.

A hexaarylbiimidazole with a reactive group which may undergo an addition or condensation reaction with a reactive group of a carrier component as described above has a general formula:

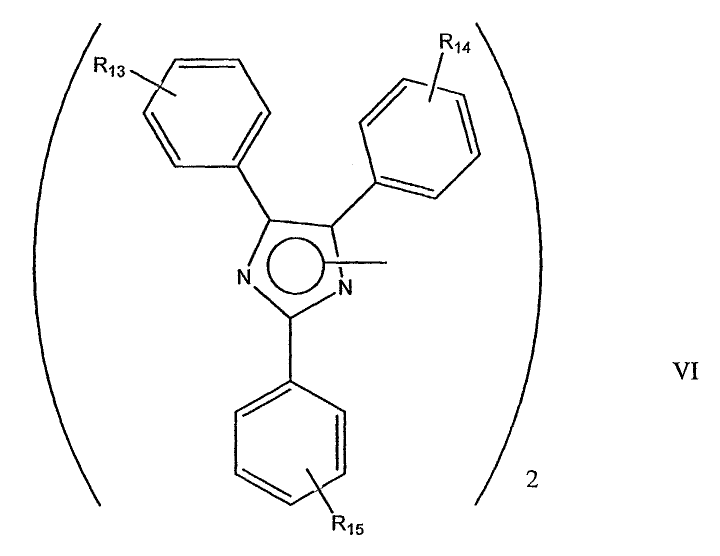

R₁₃, R₁₄ and R₁₅ are the same or different and may be a hydrogen, unsubstituted or substituted alkyl, alkoxy, unsubstituted or substituted aryl, aryloxy, hydroxyl, aminyl, carboxyl, ester, thio, isocyanate, -N(CH₂―OCH₃)₂, hydroxyalkyloxy or alkylthio. At least one of R₁₃, R₁₄ and R₁₅ is a reactive group which may react with a reactive group of the carrier compound. Such reactive groups may have a labile hydrogen, such as in hydroxyl or aminyl groups. Examples of reactive groups include, but are not limited to, ―N(CH₂―OCH₃)₂, carboxyl, ester, thio or isocyanate. Examples of spacer groups with reactive groups include, but are not limited to, (C₁ to C₁₂)hydroxyalkyl, (C₁ to C₁₂)carboxyalkyl, (C₁ to C₁₂)aminylalkyl, (C₁-C₁₂)alkylester, or hydroxyalkyloxy such as -(O-CH₂-CH₂)OH, ―(O-CH₂-CH₂-CH₂)OH and -(O-CH₂-CH₂-CH₂-CH₂)OH, (C₁ to C₁₂)aliphatic isocyanate, (C₅-C₈)cycloaliphatic isocyanate or (C₅ to C₆)aromatic isocyanate. Any aliphatic, aromatic or cycloaliphatic group with a reactive group attached to it may perform as a spacer group. Preferably, the reactive group is joined to the photoinitiator by a spacer group. A spacer is preferred because bonding the photoinitiator to the carrier component is easier and less disruptive of the photoinitiator capability to form a polymerization initiator. Preferred reactive groups are (C₁ to C₆)hydroxy alkyl or (C₁ to C₆)aminyl alkyl. In addition to R₁₅ equaling R₁₃ and R₁₄, R₁₅ also may be a halogen group such as chloro, bromo, or fluoro.

Examples of alkyl groups having 1 to 4 carbon atoms are methyl, ethyl, n-propyl, isopropyl, n-butyl, iso-butyl and test-butyl. Examples of suitable aryl groups having from 6 to 10 carbon atoms are phenyl, naphthyl, ortho-tolyl, meta-tolyl and para-tolyl. Both the alkyl and the aryl groups may be functionalized with a reactive group such as hydroxyl, aminyl or other reactive groups discussed above.

The general formula III shows structures in which two imidazole units are mutually bonded via the 1 position or the 2 position. Accordingly, the hexaarylbiimidazole compound is either one of the compounds of the following formulas VI to IX or a mixture of the two or more of such compounds.

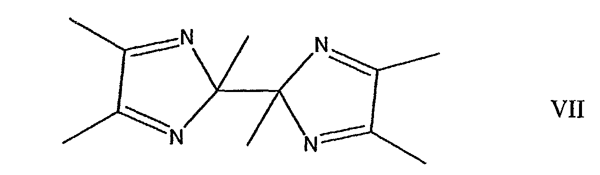

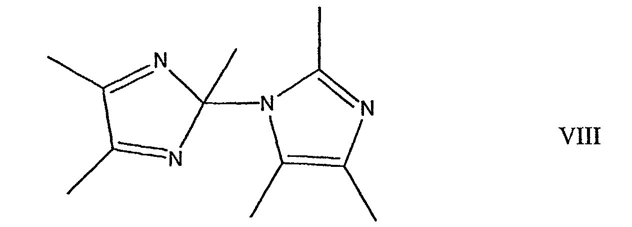

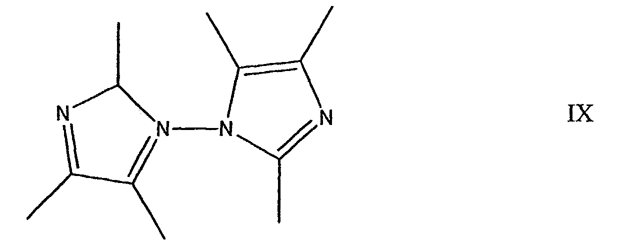

Hydrophobic photoactive compounds may be prepared by any suitable method known in the art. Hexaarylbiimidazole compounds may be prepared by oxidative coupling of triphenylimidazoles. Preparation of substituted triphenylimidazoles is described in U.S. 3,748,557, U.S. 4,311,783, and U.S. 4,622,286, the entire disclosures of which are hereby incorporated herein in their entireties by reference. In some cases, reaction mixtures in which more than one hexaarylbiimidazole is produced can be used without complete separation and purification as described in U.S. 4,622,286.

An example of forming substituted triphenylimidazoles used in the oxidation procedures to prepare the hexaarylbiimidazoles can be prepared by refluxing, in glacial acetic acid containing ammonium acetate, benzil with an appropriately substituted benzaldehyde or a benzil and benzaldehyde which are both suitably substituted, then drowning the reaction mass in water or in an ammoniun hydroxide solution, filtering and purifying the product by recrystallization; or by refluxing a benzoin and a benzaldehyde in methanol in the presence of copper acetate and ammonia; or by heating a benzil and a benzaldehyde at 180° C to 190°C in formamide as described in U.S. 3,784,557. Benzils and substituted benzils may be prepared by any suitable method in the literature such as by oxidizing corresponding benzoins as disclosed in U.S. 4,144,156, the entire disclosure of which is hereby incorporated herein in its entirety by reference.

Another method for forming a photoinitiator component with a reactive group is to react the photoinitiator, such as hexarrylbiimidazole, with an alkyl ether or ester in a Friedal Crafts reaction. The ether or ester group on the alkyl chain or spacer group attached to the photoinitiator is cleaved to get a hydroxyl group on the spacer group. The photoinitiator may then be reacted with a reactive group on a carrier component by an addition or condensation reaction to form a photoinitiator of the present invention. Such addition and condensation reactions, as well as the conditions under which they proceed, are well known in the art. Other hydrophobic photoinitiators such as benzophenones, acetophenones, anthraquinones, napthaquinones and triazine-based compounds may be functionalized with reactive groups by analogy using similar procedures.

Examples of such addition reactions that may occur include the reaction of a hydroxyl, carboxyl or aminyl reactive group on the photoinitiator or joined to the photoinitiator by a spacer group with an isocyanate pendent group on the carrier component to obtain a urethane (-NHCOO-), amide (-NHCO-) or urea bond (-NHCONH-) between the carrier component and the photoinitiator. Alternatively, an isocyanate group attached to the photoinitiator, or an isocyanate group joined to the photoinitiator by a spacer may react with a hydroxyl, carboxyl or aminyl functional group on the carrier component to form the same types of urethane, amide and urea bonds. Examples of suitable isocyanates which may be employed are described above. Ester bonds (-COOR₁₆) also may form to join a photoinitiator to a carrier such as a reaction between an acid group and an alcohol group. R₁₆ represents an organic radical. Accordingly, a water-soluble or water-emulsifiable compound containing a photoinitiator of the present invention has a general formula:

(PI)-R₁₇-CC

Where (PI) is the photoinitiator or photoactive component and CC is the carrier component with water-soluble or water-emulsifiable groups and R₁₇ is a bonding moiety which may or may not include a spacer to join the photoinitiator to the carrier component. Such bonding moieties include, but are not limited to, the urethane, amide, urea or ester bonds described above, and the spacers are the aliphatic, cycloaliphatic or aromatic radicals described earlier.

Because the photinitiator containing compounds of the present invention are water-soluble or water-emulsifiable, scum and residue formation is eliminated or reduced. Water-soluble and water-emulsifiable compounds containing photoinitiators of the present invention may be employed with conventional cross-linking agents as well as with conventional polymers used as binders in photoresists. The photoresists of the present invention compose from 20% by weight to 95% by weight of polymer binder. The polymer binder may have a photoinitiator component. The photoinitiator component also may be joined to one or more of the cross-linking agents. The compound containing the photoinitiator composes from 0.1% to 95% by weight of the photoresist, preferably from 0.1% to 25% by weight of the photoresist. The remaining weight is composed of conventional photoresist additives such as optional photoinitiators, additional cross-linking agents without the hydrophilic compound containing the photoinitiator, inert fillers, dyes, strippers or mixtures thereof. Specific amounts of the conventional additives may be added in amounts to tailor the photoresist composition to desired performance.

A wide variety of optional polymeric binders are suitable to practice the present invention. Suitable polymeric binders are generally commercially available from a variety of sources, such as Rohm and Haas Company (Philadelphia, Pennsylvania) or may be prepared by a variety of methods known in the literature. Mixtures of binders may be employed to practice the present invention. The binders may be mixed in any suitable ratio.

An example of suitable polymeric binders are those containing as polymerized units one or more ethylenically or acetylenically unsaturated monomers and one or more difunctional branch-point monomers having two polymerizable end groups and a backbone including one or more base cleavable functionalities. Suitable ethylenically or acetylenically unsaturated monomers include, but are not limited to: (meth)acrylic acid, (meth)acrylamides, alkyl (meth)acrylates, alkenyl (meth)acrylates, aromatic (meth)acrylates, vinyl aromatic monomers, nitrogen-containing compounds and their thio-analogs, substituted ethylene monomers, cyclic olefins, substituted cyclic olefins, and the like. Preferred monomers include (meth)acrylic acid, alkyl (meth)acrylates and vinyl aromatic monomers.

Typically, the alkyl (meth)acrylates useful in the present invention are (C₁-C₂₄)alkyl (meth)acrylates. Suitable alkyl (meth)acrylates include, but are not limited to, "low cut" alkyl (meth)acrylates, "mid cut" alkyl (meth)acrylates and "high cut" alkyl (meth)acrylates.

"Low cut" alkyl (meth)acrylates are typically those where the alkyl group contains from 1 to 6 carbon atoms. Suitable low cut alkyl (meth)acrylates include, but are not limited to: methyl methacrylate, methyl acrylate, ethyl acrylate, propyl methacrylate, butyl methacrylate, butyl acrylate, isobutyl methacrylate, hexyl methacrylate, cyclohexyl methacrylate, cyclohexyl acrylate and mixtures thereof.

"Mid cut" alkyl (meth)acrylates are typically those where the alkyl group contains from 7 to 15 carbon atoms. Suitable mid cut alkyl (meth)acrylates include, but are not limited to: 2-ethylhexyl acrylate ("EHA"), 2-ethylhexyl methacrylate, octyl methacrylate, decyl methacrylate, isodecyl methacrylate (based on branched (C₁₀)alkyl isomer mixture), undecyl methacrylate, dodecyl methacrylate (also known as lauryl methacrylate), tridecyl methacrylate, tetradecyl methacrylate (also known as myristyl methacrylate), pentadecyl methacrylate and mixtures thereof. Particularly useful mixtures include dodecyl-pentadecyl methacrylate, a mixture of linear and branched isomers of dodecyl, tridecyl, tetradecyl and pentadecyl methacrylates; and lauryl-myristyl methacrylate.

"High cut" alkyl (meth)acrylates are typically those where the alkyl group contains from 16 to 24 carbon atoms. Suitable high cut alkyl (meth)acrylates include, but are not limited to: hexadecyl methacrylate, heptadecyl methacrylate, octadecyl methacrylate, nonadecyl methacrylate, cosyl methacrylate, eicosyl methacrylate and mixtures thereof. Particularly useful mixtures of high cut alkyl (meth)acrylates include, but are not limited to: cetyl-eicosyl methacrylate, which is a mixture of hexadecyl, octadecyl, cosyl and eicosyl methacrylate; and cetyl-stearyl methacrylate, which is a mixture of hexadecyl and octadecyl methacrylate.

The mid-cut and high-cut alkyl (meth)acrylate monomers described above are generally prepared by standard esterification procedures using technical grades of long chain aliphatic alcohols, and these commercially available alcohols are mixtures of alcohols of varying chain lengths containing between 10 and 15 or 16 and 20 carbon atoms in the alkyl group. Examples of these alcohols are the various Ziegler catalyzed ALFOL alcohols from Vista Chemical company, i.e., ALFOL 1618 and ALFOL 1620, Ziegler catalyzed various NEODOL alcohols from Shell Chemical Company, i.e. NEODOL 25L, and naturally derived alcohols such as Proctor & Gamble's TA-1618 and CO-1270. Consequently, for the purposes of this invention, alkyl (meth)acrylate is intended to include not only the individual alkyl (meth)acrylate product named, but also to include mixtures of the alkyl (meth)acrylates with a predominant amount of the particular alkyl (meth)acrylate named.

The alkyl (meth)acrylate monomers useful in the present invention may be a single monomer or a mixture having different numbers of carbon atoms in the alkyl portion. Also, the (meth)acrylamide and alkyl (meth)acrylate monomers useful in the present invention may optionally be substituted. Suitable optionally substituted (meth)acrylamide and alkyl (meth)acrylate monomers include, but are not limited to: hydroxy(C₂-C₆)alkyl (meth)acrylates, dialkylamino(C₂-C₆)-alkyl (meth)acrylates, dialkylamino(C₂-C₆)alkyl (meth)acrylamides.

Other substituted (meth)acrylate and (meth)acrylamide monomers useful in the present invention are those with a dialkylamino group or dialkylaminoalkyl group in the alkyl radical. Examples of such substituted (meth)acrylates and (meth)acrylamides include, but are not limited to: dimethylaminoethyl methacrylate, dimethylaminoethyl acrylate, N,N-dimethylaminoethyl methacrylamide, N,N-dimethyl-aminopropyl methacrylamide, N,N-dimethylaminobutyl methacrylamide, N,N-di-ethylaminoethyl methacrylamide, N,N-diethylaminopropyl methacrylamide, N,N-diethylaminobutyl methacrylamide, N-(1,1-dimethyl-3-oxobutyl) acrylamide, N-(1,3-diphenyl-1-ethyl-3-oxobutyl) acrylamide, N-(1-methyl-1-phenyl-3-oxobutyl) methacrylamide, and 2-hydroxyethyl acrylamide, N-methacrylamide of aminoethyl ethylene urea, N-methacryloxy ethyl morpholine, N-maleimide of dimethylaminopropylamine and mixtures thereof.

Other substituted (meth)acrylate monomers useful in the present invention are silicon-containing monomers such as γ-propyl tri(C₁-C₆)alkoxysilyl (meth)acrylate, γ-propyl tri(C₁-C₆)alkylsilyl (meth)acrylate, γ-propyl di(C₁-C₆)alkoxy(C₁-C₆)alkylsilyl (meth)acrylate, γ-propyl di(C₁-C₆)alkyl(C₁-C₆)alkoxysilyl (meth)acrylate, vinyl tri(C₁-C₆)alkoxysilyl (meth)acrylate, vinyl di(C₁-C₆)alkoxy(C₁-C₆)alkylsilyl (meth)acrylate, vinyl (C₁-C₆)alkoxydi(C₁-C₆)alkylsilyl (meth)acrylate, vinyl tri(C₁-C₆)alkylsilyl (meth)acrylate, 2-propylsilsesquioxane (meth)acrylate and mixtures thereof.

The vinyl aromatic monomers useful as unsaturated monomers in the present invention include, but are not limited to: styrene, hydroxystyrene, α-methylstyrene, vinyltoluene, *p*-methylstyrene, ethylvinylbenzene, vinylnaphthalene, vinylxylenes, and mixtures thereof. The vinylaromatic monomers also include their corresponding substituted counterparts, such as halogenated derivatives, i.e., containing one or more halogen groups, such as fluorine, chlorine or bromine; and nitro, cyano, (C₁-C₁₀)alkoxy, halo(C₁-C₁₀)alkyl, carb(C₁-C₁₀)alkoxy, carboxy, amino, (C₁-C₁₀)alkylamino derivatives and the like.

The nitrogen-containing compounds and their thio-analogs useful as unsaturated monomers in the present invention include, but are not limited to: vinylpyridines such as 2-vinylpyridine or 4-vinylpyridine; (C₁-C₈)alkyl substituted N-vinyl pyridines such as 2-methyl-5-vinyl-pyridine, 2-ethyl-5-vinylpyridine, 3-methyl-5-vinylpyridine, 2,3-dimethyl-5-vinyl-pyridine, and 2-methyl-3-ethyl-5-vinylpyridine; methyl-substituted quinolines and isoquinolines; N-vinylcaprolactam; N-vinylbutyrolactam; N-vinylpyrrolidone; vinyl imidazole; N-vinyl carbazole; N-vinyl-succinimide; (meth)acrylonitrile; *o-, m-,* or *p*-aminostyrene; maleimide; N-vinyl-oxazolidone; N,N-dimethyl aminoethyl-vinyl-ether; ethyl-2-cyano acrylate; vinyl acetonitrile; N-vinylphthalimide; N-vinyl-pyrrolidones such as N-vinyl-thio-pyrrolidone, 3 methyl-1-vinyl-pyrrolidone, 4-methyl-1-vinyl-pyrrolidone, 5-methyl-1-vinyl-pyrrolidone, 3-ethyl-1-vinyl-pyrrolidone, 3-butyl-1-vinyl-pyrrolidone, 3,3-dimethyl-1-vinyl-pyrrolidone, 4,5-dimethyl-1-vinyl-pyrrolidone, 5,5-dimethyl-1-vinyl-pyrrolidone, 3,3,5-trimethyl-1-vinyl-pyrrolidone, 4-ethyl-1-vinyl-pyrrolidone, 5-methyl-5-ethyl-1-vinyl-pyrrolidone and 3,4,5-trimethyl-1-vinyl-pyrrolidone; vinyl pyrroles; vinyl anilines; and vinyl piperidines.

The substituted ethylene monomers useful as unsaturated monomers is in the present invention include, but are not limited to: vinyl acetate, vinyl formamide, vinyl chloride, vinyl fluoride, vinyl bromide, vinylidene chloride, vinylidene fluoride, vinylidene bromide, tetrafluoroethylene, trifluoroethylene, trifluoromethyl vinyl acetate, vinyl ethers and itaconic anhydride.

Suitable cyclic olefin monomers useful in the present invention are (C₅-C₁₀)cyclic olefins, such as cyclopentene, cyclopentadiene, dicylopentene, cyclohexene, cyclohexadiene, cycloheptene, cycloheptadiene, cyclooctene, cyclooctadiene, norbornene, maleic anhydride and the like. Such cyclic olefins also include spirocyclic olefin monomers such as spirocyclic norbornenyl monomers, spirocyclic cyclohexene monomers, spirocyclic cyclopentene monomers and mixtures thereof. Suitable substituted cyclic olefin monomers include, but are not limited to, cyclic olefins having one or more substituent groups selected from hydroxy, aryloxy, halo, (C₁-C₁₂)alkyl, (C₁-C₁₂)haloalkyl, (C₁-C₁₂)hydroxyalkyl, (C₁-C₁₂)halohydroxyalkyl such as (CH₂)_{n"}C(CF₃)₂OH where n" = 0 to 4, (C₁-C₁₂)alkoxy, thio, amino, (C₁-C₆)alkylamino, (C₁-C₆)dialkylamino, (C₁-C₁₂)alkylthio, carbo(C₁-C₂₀)alkoxy, carbo(C₁-C₂₀)haloalkoxy, (C₁-C₁₂)acyl, (C₁-C₆)alkylcarbonyl(C₁-C₆)alkyl, and the like. Particularly suitable substituted cyclic olefins include maleic anhydride and cyclic olefins containing one or more of hydroxy, aryloxy, (C₁-C₁₂)alkyl, (C₁-C₁₂)haloalkyl, (C₁-C₁₂)hydroxyalkyl, (C₁-C₁₂)halohydroxyalkyl, carbo(C₁-C₂₀)alkoxy, and carbo(C₁-C₂₀)haloalkoxy. It will be appreciated by those skilled in the art that the alkyl and alkoxy substituents may be optionally substituted, such as with halogen, hydroxyl, cyano, (C₁-C₆)alkoxyl, mercapto, (C₁-C₆)alkylthio, amino, and the like.

Any of a wide variety of difunctional branch-point monomers are suitable for use in preparing the branched binder polymers of the present invention provided that such branch-point monomers contain a backbone comprising one or more base cleavable functionalities or moieties, where such functionalities are disposed between the polymerizable groups of the branch-point monomer. By "base cleavable functionality" is meant any functionality or group that can be cleaved by a base such as hydroxide ion, alkoxide ion, ammonia, amines and the like.

A wide variety of difunctional branch-point monomers containing base cleavable moieties may be used in the present invention. In general, such branch-point monomers have the structure

A"-Z'-B'

where A" and B' each include one or more polymerizable groups, and Z' includes one or more base cleavable groups. Suitable polymerizable groups for A" and B' include, but are not limited to, isocyanate ("-NCO"), R₁₈R₁₉C=CR-, R₁₈-C=C-, R₁₈R₁₉C=CR₂₀C(O)-O-, R₁₈R₁₉C=CR₂₀-O-, and -C(O)-O-R₂₁; wherein R₁₈, R₁₉ and R₂₀ are independently selected from H, (C₁-C₄)alkyl and halo; R₂₁ is selected from H, (C₁-C₄)alkyl, and NR₂₂R₂₃; and R₂₂ and R₂₃ are independently selected from H and (C₁-C₄)alkyl. In addition to one or more base cleavable groups, the group Z' may optionally include one or more spacer groups. Z' may suitably have the general formula Sₓ₄(BCG)_{y4}; wherein S is a spacer group; (BCG) is a base cleavable group; x₄ = 0-20 and y₄ = 1-30. It is preferred that y = 2-20. Suitable spacer groups include, but are not limited to, alkyleneoxy, aryleneoxy, (C₁-C₂₀)alkylene, substituted(C₁-C₂₀)alkylene, (C₆-C₂₀)aralkylene, substituted (C₆-C₂₀)aralkylene, and the like. Suitable alkyleneoxy groups have the general formula (-CHR₂₄-CH₂₀-)ₙ₃, (-OCHR₂₄-CH₂-)ₘ₃, or (-O-CH₂-CH₂-CH₂-)ₚ₃, where R₂₄ is H or CH₃, and n₃, m₃ and p₃ are each 1-1000. Exemplary alkylenoxy groups include ethyleneoxy, propyleneoxy and ethyleneoxy/propyleneoxy mixtures. Aryleneoxy or arylene ether spacers include phenyleneoxy (phylene ether) spacers having the general formula (-C₆H₄-O-)_{z3} where z₃ = 1-1000, biphenylene ethers, phenanthryl ethers, naphthyl ethers, and mixtures thereof. When two or more spacer groups are used, they may be the same or different.

"Substituted alkyl" refers to any alkyl group having one or more of its hydrogens replaced by another substituent group selected from halo, cyano, hydroxyl, (C₁-C₈)alkoxy, amino, (C₁-C₆)alkylamino, di(C₁-C₆)alkylamino, phenyl, carb(C₁-C₆)alkoxy, and the like. Likewise, "substituted aralkyl" refers to any aralkyl group having one or more of its hydrogens replaced by another substituent group selected from halo, cyano, hydroxyl, (C₁-C₈)alkoxy, amino, (C₁-C₆)alkylamino, di(C₁-C₆)alkylamino, phenyl, carb(C₁-C₆)alkoxy, and the like.

Such spacer groups may be selected to provide additional properties. For example, alkyleneoxy spacers, such as ethyleneoxy and/or propyleneoxy moieties, may help to emulsify the polymeric binders for use in water borne photoresists. Spacers having extended chain length may also provide improved flexibility and be particularly useful in conformal photoresist formulations. The choice of such spacer groups depend upon the particular use of the polymer and the other components in the formulation, and is within the ability of one skilled in the art.

Any base cleavable group is suitable for use in Z', but is preferably selected from anhydrides (-C(O)-O-(O)C-), esters (-C(O)-O-), carbonates, sulfonyl esters (-SO₂-O-) and the like, and more preferably esters. It is more preferred that the difunctional branch-point monomers contain 2 or more base cleavable groups and still more preferably 3 or more base cleavable groups. Particularly suitable difunctional branch-point monomers contain 4 base cleavable groups, and more particularly 4 or more ester linkages. It is further preferred that the difunctional branch point monomer contain as polymerizable end groups moieties that also contain one or more base cleavable functionalities, such as (meth)acrylate esters. When the difunctional branch-point monomers contain 2 or more base cleavable groups, such groups may be directly bonded to each other or may be separated by one or more spacer groups. An exemplary structure for such branch-point monomers having multiple base cleavable groups is A'(S1)ₓ₁-(BCG)1-(S2)ₓ₂-(BCG)2 -(S3)ₓ₃-B', wherein S1, S2 and S3 refer to spacer groups 1-3, respectively, (BCG) 1 and (BCG)2 refer to base cleavable groups 1 and 2, respectively, x 1 + x2 + x3 = 0-20, and A', B', S, (BCG) and B' are as defined above. Other suitable structures having more or fewer spacers and/or base cleavable groups or different configurations of such groups are well within the ability of those skilled in the art.

Suitable difunctional branch-point monomers useful in preparing the branched binder polymers of the present invention include, but are not limited to, acrylic anhydride, methacrylic anhydride, and ester linkage containing monomers having (meth)acrylate end groups. Exemplary difunctional branch-point monomers including one or more urethane linkages and having (meth)acrylate end groups are: pdmbi-pcp0200-pdmbi, pdmbi-pcp0201-pdmbi, pdmbi-pcp0230-pdmbi, eh6cl4-hdi-ppg1000-hdi-eh6cl4, eh6cl4-hdi-pcp0230-hdi-eh6cl4, eh6cl4-hdi-ppg425-hdi-dmpa-hdi-ppg425-hdi-eh6cl4, 2hema-hdi-pcp0230-hdi-ppg425-hdi-pcp0230-hdi-2hema, 2hema-hdi-pcp0230-hdi-peg400-hdi-pcp0230-hdi-2hema, 2hema-hdi-pcp0200-hdi-pcp0230-hdi-pcp0200-hdi-2hema, e6hem-hdi-pcp0200-hdi-pcp0230-hdi-pcp0200-hdi-e6hem, e6hem-hdi-pcp0200-hdi-ppg1000-hdi-pcp0200-hdi-e6hem, e6hem-hdi-ppg425-hdi-pcp0230-hdi-ppg425-hdi-e6hem, e6hem-hdi-ppg1000-hdi-pcp0230-hdi-ppg1000-hdi-e6hem, e6hem-hdi-pcp0230-hdi-ppg425-hdi-pcp0230-hdi-e6hem, and e6hem-hdi-ppg1000-hdi-pcp0201-hdi-ppg1000-hdi-e6hem. In the above described difunctional branch-point monomers, each "dash" represents a urethane group (formed when an isocyanate group reacts with a hydroxyl group) between the adjacent moieties. Such urethane linkages are not required in the present branch-point monomers. The abbreviations for the moieties are: hdi = 1,6-hexamethylene diisocyanate; pcp0200 = TONE™ Polyol 0200 Diol (containing carboxylic ester groups); pcp0201 = TONE™ Polyol 0201 Diol (contains carboxylic ester groups); pcp0230 = TONE™ Polyol 0230 Diol (contains carboxylic ester groups); ppg425 = polypropylene glycol having a molecular weight of approximately 425; ppg1000 = polypropylene glycol having a molecular weight of approximately 1000; dmpa = dimethylolpropionic acid; pdmbi = 3-isopropenyl-alpha,alpha-dimethylbenzyl isocyanate; 2hema = 2-hydroxyethyl methacrylate (contains ester group and a polymerizable end group); e6hem = ethoxylated hydroxyethyl methacrylate (contains ester group and a polymerizable end group); and eh6cl4 = ethoxylated caprolactone-derived methacrylate (contains ester groups and a polymerizable end group). Such branch-point monomers are generally commercially available or may be readily prepared by known methods. TONE™ is a trademark for polycaprolactone diols, available from the Dow Chemical Company (Midland, Michigan). Other suitable polycaprolactone diols are available from Solvay under the CAPA brand name. Typically, the molecular weight of the branch-point monomers is ≥ 450, and preferably from 450 to 6000.

The branched polymeric binders of the present invention include as polymerized units one or more difunctional branch-point monomers having two polymerizable end groups and a backbone including one or more base cleavable functionalities. When polymeric binders are prepared from tri-, tetra- or higher-functional branch-point monomers, i.e. those containing 3 or more polymerizable end groups, such polymeric binders are much more likely to suffer from gel formation, which makes such binders unsuitable for use in photoresist compositions. Further, when polymeric binders are prepared from relatively low molecular weight, i.e. < 450, difunctional branch-point monomers containing no urethane linkages and containing (meth)acrylate esters as both polymerizable end groups, such polymeric binders also suffer from gel formation. Such gel formation is not a problem when the difunctional branch-point monomers are higher molecular weight, i.e. ≥ 450, monomers containing (meth)acrylate esters as both polymerizable end groups, or when such monomers contain one or more urethane linkages.

The present invention further provides a compound having the formula A"-Z'-B'wherein A" and B' each include one or more polymerizable groups and Z' includes one or more base cleavable groups.

More than one difunctional branch-point monomer may be used to prepare the branched binder polymers. Thus, mixtures of difunctional branch-point monomers may advantageously be used in the present invention. Typically, the total amount of such difunctional branch-point monomers in the branched binder polymers is from 0.1 to 100 wt% based upon the total weight of the monomers used to prepare the binder polymer, preferably from 0.1 to 25 wt%, and more preferably from 0.1 to 10 wt%.

The branched binder polymers may be prepared by a variety of methods known in the art, such as free radical polymerization. Preferably, the difunctional branched polymeric binders contain sufficient acid functionality to render the binder polymers soluble and removable upon development. The term "acid functionality" refers to any functionality capable of forming a salt upon contact with alkaline developer, such as dilute alkaline aqueous sodium or potassium hydroxide, e.g. 1 to 3 wt% solutions. Suitable acid functionality includes, but is not limited to, carboxylic acids, sulfonic acids, phosphonic acids and phenols. In general, the binder polymers have an acid number of up to about 250, preferably up to about 200. Typical ranges of acid numbers are from 15 to 250 and preferably from 50 to 250. Such acid numbers are based on the amount of KOH (potassium hydroxide) in mg to neutralize 1 g (dry weight) of binder polymer.

While the oligomers or monomers with the photoinitiator component of the present invention may be the only cross-linking agent in the photoresist, other monomers may be added which polymerize into a network around the polymeric binders. A wide variety of monomers may be used. Suitable monomers include, but are not limited to: methyl acrylate, 2-ethylhexyl acrylate, n-butyl acrylate, n-hexyl acrylate, methyl methacrylate, hydroxyethyl acrylate, butyl methacrylate, octyl acrylate, 2-ethoxyethyl methacrylate, t-butyl acrylate, 1,5-pentanediol diacrylate, N,N-diethylaminoethyl acrylate, ethylene glycol diacrylate, 1,3-propanediol diacrylate, decamethylene glycol diacrylate, decamethylene glycol dimethacrylate; 1,4-cyclohexanediol diacrylate, 2,2-dimethylol propane diacrylate, glycerol diacrylate, tripropylene glycol diacrylate, glycerol triacrylate, 2,2-di(p-hydroxyphenyl)-propane dimethacrylate, triethylene glycol diacrylate, polyoxyethyl-2-2-di(p-hydroxyphenyl)-propane dimethacrylate, triethylene glycol dimethacrylate, polyoxypropyltrimethylol propane triacrylate, ethylene glycol dimethacrylate, butylene glycol dimethacrylate, 1,3-propanediol dimethacrylate, butylene glycol dimethacrylate, 1,3-propanediol dimethacrylate, 1,2,4-butanetriol trimethacrylate, 2,2,4-trimethyl-1,3-pentanediol dimethacrylate, pentaerythritol trimethacrylate, 1-phenyl ethylene-1,2-dimethacrylate, pentaerythritol tetramethacrylate, trimethylol propane trimethacrylate, 1,5-pentanediol dimethacrylate, and 1,4-benzenediol dimethacrylate; styrene and substituted styrene, such as 2-methyl styrene and vinyl toluene and vinyl esters, such as vinyl acrylate and vinyl methacrylate. Such monomers may be included in conventional amounts.

Although monomers, oligomers and polymers of the present invention have a photoinitiator component, photoimageable compositions of the present invention optionally may contain an additional photoactive agent. Optional photoactive agents useful in the present invention may be photoacid generators, photobase generators or free-radical generators. The present photoimageable compositions may be positive-acting or negative-acting, and preferably are negative-acting. Mixtures of photoactive agents allow the photoactivity of the compositions to be tailored to specific applications.

Suitable photoacid generators include halogenated triazines, onium salts, sulfonated esters, halogenated sulfonyloxy dicarboximides, diazodisulfones, α-cyanooxyaminesulfonates, imidesulfonates, ketodiazosulfones, sulfonyldiazoesters, 1,2-di(arylsulfonyl)hydrazines and the like. Particularly useful halogenated triazines include halomethyl-s-triazines.

Suitable free-radical generators include, but are not limited to, n-phenylglycine, aromatic ketones such as benzophenone, N, N'-tetramethyl-4, 4'-diaminobenzophenone [Michler's ketone], N,N'-tetraethyl-4,4'-diaminobenzophenone, 4-methoxy-4'-dimethylaminobenzophenone, 3,3'-dimethyl-4-methoxybenzophenone, p,p'-bis(dimethylamino)benzophenone, p,p'-bis(diethylamino)-benzophenone, anthraquinone, 2-ethylanthraquinone, naphthaquinone and phenanthraquinone, benzoins such as benzoin, benzoinmethylether, benzoinethylether, benzoinisopropylether, benzoin-n-butylether, benzoin-phenylether, methylbenzoin and ethybenzoin, benzyl derivatives such as dibenzyl, benzyldiphenyldisulfide and benzyldimethylketal, acridine derivatives such as 9-phenylacridine and 1,7-bis(9-acridinyl)heptane, thioxanthones such as 2-chlorothioxanthone, 2-methylthioxanthone, 2,4-diethylthioxanthone, 2,4-dimethylthioxanthone and 2-isopropylthioxanthone, acetophenones such as 1,1-dichloroacetophenone, p-t-butyldichloro-acetophenone, 2,2-diethoxyacetophenone, 2,2-dimethoxy-2-phenylacetophenone, and 2,2-dichloro-4-phenoxyacetophenone, 2,4,5-triarylimidazole dimers such as 2-(o-chlorophenyl)-4,5-diphenylimidazole dimer, 2-(o-chlorophenyl)-4,5-di(m-methoxyphenyl imidazole dimer, 2-(o-fluorophenyl)-4,5-diphenylimidazole dimer, 2-(o-methoxyphenyl)-4,5-diphenylimidazole dimer, 2-(p-methoxyphenyl)-4,5-diphenylimidazole dimer, 2,4-di(p-methoxyphenyl)-5-phenylimidazole dimer, 2-(2,4-dimethoxyphenyl)-4,5-diphenylimidazole dimer and 2-(p-methylmercaptophenyl)-4,5-diphenylimidazole dimer, and the like. Though, not a free-radical generator, triphenylphosphine may be included in the photoactive chemical system as a catalyst. Such free-radical generators are particularly suitable for use with negative-acting photoimageable compositions, and particularly suitable for use with negative-acting dry film photoimageable compositions of the present invention.

Because the photoresists of the present invention have monomers, oligomers or polymers with a photoinitiator component, optional photoactive agents are added in amounts from 0.1 to 2 wt%, and more preferably from 0.1 to 0.5 wt% of the photoresist.

The present photoimageable compositions may be solvent-borne or water-borne. Whether such compositions are solvent- or water-borne depends upon the choice of polymer binder, including the choice of monomers and difunctional branch-point monomers used to prepare the polymer binders. Such choices of monomers and difunctional branch-point monomers is well within the ability of one skilled in the art. Thus, the present photoimageable compositions may optionally contain water, a solvent or a water-solvent mixture. Suitable solvents include, but are not limited to: ketone solvents such as acetone, methyl ethyl ketone, cyclohexanone, methyl isoamyl ketone and 2-heptanone; polyhydric alcohols and derivatives thereof such as ethyleneglycol, ethyleneglycol monoacetate, diethyleneglycol, diethyleneglycol monoacetate, propyleneglycol, propyleneglycol monoacetate, dipropyleneglycol and dipropyleneglycol monoacetate as well as monomethyl, monoethyl, monopropyl, monobutyl and monophenyl ethers thereof; cyclic ether solvents such as dioxane; ester solvents such as methyl lactate, ethyl lactate, methyl acetate, ethyl acetate, butyl acetate, methyl pyruvate, ethyl pyruvate, methyl methoxypropionate and ethyl ethoxypropionate; and amide solvents such as N,N-dimethyl formamide, N,N-dimethyl acetamide, N-methyl-2-pyrrolidone, 3-ethoxyethyl propionate, 2-heptanone, γ-butyrolactone, and mixtures thereof.

Other optional additives that may be used in the present photoimageable compositions include, but are not limited to: plasticizers, rheology modifiers, fillers, dyes, film forming agents, strip enhancers such as hydrophobic trihalomethyl containing photoresist strip enhancers or mixtures thereof. Suitable plasticizers include esters such as dibenzoate esters or urethanes. Suitable hydrophobic trihalomethyl containing photoresist strip enhancers include a wide variety of compounds containing a trihalomethyl group which hydrolyzes to carboxylate anions during stripping of the photoresist. Preferably, such hydrophobic trihalomethyl containing photoresist strip enhancer is alpha-trichloromethyl benzyl acetate. Such optional additives may be present in various concentrations in a photoresist composition. For example, fillers and dyes may be used in relatively large concentrations, e.g. in amounts of from about 5 to 30 percent by weight, based on the total weight of the composition's dry components.

Photoresist compositions of the present invention may be prepared by combining the monomers, oligomers and polymers of the present invention with one or more optional solvents and optional additives in any order.

Processing of the photoimageable or photoresist compositions may be in any conventional manner. For example, a photoresist layer, either formed from a liquid composition or transferred as a layer from a dry film, is applied to a substrate. When a liquid photoresist composition is used, it may be applied to a substrate by any known means, such as spinning, dipping, roller coating and the like.

The photoresist compositions may be used on a variety of substrates used in the manufacture of electronic devices such as printed wiring boards and integrated circuits. Suitable substrates include copper surfaces of copper clad boards, printed wiring board inner layers and outer layers, wafers used in the manufacture of integrated circuits and the like.

Once the photoresist is applied to the substrate, it is imaged or exposed to actinic radiation through appropriate artwork. Upon exposure to actinic radiation, a radical is believed to be generated from the photoactive component of the present invention. In the case of a negative-acting photoresist, exposure of actinic radiation polymerizes the monomer or oligomer in exposed areas, resulting in a cross-linked structure that is resistant to developer. Next, the uncured photoresist is developed such as by using dilute alkaline aqueous solution. Suitable developers include 1-3 wt% aqueous solutions of sodium hydroxide or potassium hydroxide, or 0.5-1 wt% sodium or potassium carbonate. Organic based developers, such as tetraalkylammonium hydroxide based developers, may be used but are less preferred. During such development, acidic groups of the binder polymers form salts which render the binder polymers soluble and removable. Thus, the present invention provides a method for forming a relief image including the steps of: a) disposing on a printed wiring board substrate a photoresist composition which includes a monomer, oligomer, or polymer which contains a photoactive component and any optional additives; b) imaging the photoresist; and c) developing the photoresist.

In the case of negative-acting photoresists applied to copper surfaces of copper clad boards, an etchant may be used after development to remove copper from those areas where the photoresist was removed, thereby forming a printed circuit. The remaining resist is then removed using a stripper.

The present invention further provides a method of manufacturing a printed wiring board including the steps of: a) disposing on a printed wiring board substrate a photoresist composition which includes a water-soluble or water-emulsifiable compound containing a photoinitiator and any optional components; b) imaging the photoresist; and c) developing the photoresist.

The present photoresist compositions may show enhanced removal as compared to conventional photoresists. Thus, the present invention also provides a method of enhancing the removal of a photoresist composition from a substrate including the step of combining monomers, oligomers, or polymers having a photoinitiator component and any optional components to form a photoresist composition; disposing the photoresist composition of a substrate; imaging the photoresist composition; and developing the imaged photoresist composition. In conventional photoresists, the photoresist is stripped by action of a base on the polymerized monomer network, which is typically cleaved by the base.

In addition to reducing or eliminating residue and scum formation from uncured photoresist, photoresist compositions also show good adhesion and good stripping with substantially no loss of chemical resistance. Typically, as adhesion of a dry film photoresist is improved, the photoresist composition is harder to strip. The present photoresist compositions surprisingly provide both good adhesion and good stripping.

### Example 1

To 2.1 parts of hydroxybutyl substituted benzil (0.01 mole) dissolved in 50 parts of glacial acetic acid containing 6 parts of ammonium acetate (0.078 mole) is added 1.4 parts of o-chlorobenzaldehyde (0.01 mole), and the solution is refluxed for 2 hours. The solution is then drowned in 200 parts of cold water whereupon 3.1 parts of reaction product precipitate. The product is isolated by filtration and purified by recrystalling twice from ethanol. The product, a chlorinated hydroxybutyl imidazole, is a white crystalline solid.

To 1.1 parts of the above prepared imidazole dissolved in 100 parts of ethanol containing 12 parts of potassium hydroxide is added 450 parts of a 1% by weight water solution of potassium ferricyanide at a rate of 5 parts per minute for 1.5 hours with continuous stirring. The oxidation reaction product in an amount of 1.0 parts precipitates from the reaction mixture, is isolated by filtration, and is washed with water until free from ferricyanide. The product is dried at 56° C. for eight hours at 0.1 mm. mercury pressure after predrying overnight in a vacuum oven at 50° C. It is solvated with two moles of enthanol for every three moles of biimidazole. The product is a biimidazole having the general formula shown below.

A portion of the ethanol-solvated product was dried azeotropically with cyclohexane to produce non-solvated material. Recrystallization from ether also yields the non-solvated product.

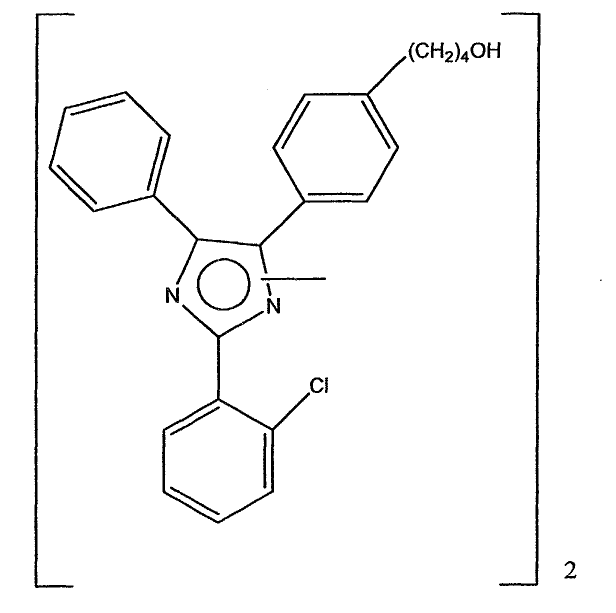

The above photoactive component is then reacted with the pendent carboxyl groups of an oligomer composed of methylmethacrylate and acrylic acid to bind the photoactive component with the oligomer by an ester linkage. The oligomer is prepared by free radical polymerization of the methylmethacrylate and acrylic acid monomers. The methylmethacrylate and acrylic acid monomers are obtainable from Rohm and Haas Company. The reaction takes place in an aqueous acidic environment. The reactants are refluxed in the acidic environment from 90° C to 100° C to obtain a water-soluble photoinitiator yield of from 85% to 90% by weight.

The hydrophilic photoinitiator containing compound may be employed as a cross-linking agent or further polymerized and employed as a polymer binder in a photoresist formulation. Upon exposure to light, a radical from the photoactive component is generated to polymerize the cross-linking agents of the formulation.

### Example 2

### Negative-Acting Photoresists

The following ingredients are blended together in the given proportions to provide a negative-acting photoresist composition of the present invention.

| Formulation | |
|---|---|
| Ingredient | Percent by Weight |
| Acrylic copolymer binder¹ | 40 |
| Caprolactone 2 hydroxyethyl methacrylate | 5 |
| Trimethylol propane triacrylate (TMPTA) | 15 |
| photoinitiator containing compound² | 5 |
| bis(dialkylaminophenyl) ketone | 0.04 |
| tris(dialkylaminophenyl) methane | 0.3 |
| aromaticsulfonamide | 3.5 |
| modifideacridine | 0.2 |

| | |
|---|---|
| Table Footnotes ¹ 88,000-91,000 Mw copolymer of methyl methacrylate, methacrylic acid, n-butyl acrylate, Tg 90°C., 150 acid number. | |
| ² Photoinitiator of Example 1. | |

A mixture is prepared at about 70% solids in 2-butanone and coated onto a 0.8 mil polyester carrier film and dried to approximately 1% residual VOC's. A thin film of about 1.5 mils thickness is obtained. The films are then laminated at 121° C., 40 psi, 1 meter per minute, onto chemically cleaned 1 oz. copper/0.059 FR-4/1 oz. clad copper laminate and imaged on a 5 kw printer through a silver halide phototool with an adjusted exposure to obtain a copper step of 9 as measured with a Stouffer® 21 step wedge. The panels are then developed in 1% sodium carbonate monohydrate at 29° C. to remove the photoresist in the unexposed portions followed by several spray rinses using tap water and the deionized water. The imaged board is then etched in 2N cupric chloride/HCl at 45° C. The etched boards are then stripped of the imaged and developed photoresist in a 3% sodium hydroxide solution at 49° C., followed by a spray rinse of tap water.

### Example 3

A radiation curable solder mask composition is prepared in two parts as follows:

| | Percent By Weight |
|---|---|
| Component A | |
| Esterified styrene-maleic anhydride copolymer¹ | 25.0 |
| Photoinitiator containing compound² | 5.0 |
| Multifunctional (meth)acrylate³ | 7.5 |
| Pigment | 4.0 |
| Flow promoter | 3.5 |
| Anti-abrasion agent | 3.5 |
| Air release agent | 3.5 |
| Filler | 17.5 |
| Inert diluent | 10.5 |

| Component B | |
|---|---|
| Multifunctional (meth)acrylate³ | 25.0 |
| Multifunctional epoxy⁴ | 30.0 |
| Thermal cross-linking agent⁵ | 7.5 |
| Pigment/filler | 12.5 |
| Inert diluent | 25.0 |

| | |
|---|---|
| ¹ Pro 1100, Sartomer Co., Exton, Pa. | |
| ² Photoinitiator of Example 1 | |
| ³ SR 351, Sartomer Co. | |
| ⁴ ECN1299, CibaGeigy Co. (Resin Division) | |
| ⁵ Dyhard 100S, SKW Inc. | |

Component A and Component B are mixed, in a ratio of 3:1 at room temperature, and the composition so produced is screen-printed onto printed circuit boards using a 70 Durometer squeegee. The boards are then heat treated at 160° F. for various lengths of time to determine the operating window for pre-baking. The pre-baked boards are then subjected to development using a 10 g/L solution of potassium carbonate at 25°-30° C. for 40 seconds. Boards are baked for 50 minutes and exhibit clean removal of the composition with no residual scum is remaining on the board.

Additional printed circuit boards are coated with the composition in the same manner as above, the composition is dried at 70° C. for twenty minutes, cooled to room temperature, and then identically processed to coat the other side of the board (70° C. drying for 40 minutes). Negatives are brought into contact with the coatings, and each coating is then subjected to 150 millijoules of ultraviolet radiation. The coatings are developed using potassium carbonate solution, 25°-30° C. for 40 seconds. The remaining imagewise distribution of photopolymer is then given a post-exposure of 2-4 joules, and then baked for 1 hour at 150° C.

The so-treated coating is tested for flexibility using the cross-hatch razor technique in which several intersecting lines are cut into the coating. The coating is found to be flexible, with no loss of adhesion.

### Example 4

The photoimageable composition of Example 2 is applied to a polyester support sheet and dried. A polyethylene protective sheet is applied to the side of the photoimageable composition opposite the polyester support sheet. A thin film of 1.4 mil thickness is obtained. The polyethylene sheet is removed and the dried film with support sheet is laminated to a copper-clad board using a hot roll laminator. The roll temperature is 122° C.; the roll speed is one meter per minute; and the roll pressure is 2.8 bars. The polyester support sheet is removed, and artwork is laid directly on the photoimageable composition layer. The photoimageable composition is exposed to 81 mJ/cm² actinic radiation through the artwork. After removal of the artwork, the photoimageable composition is developed in 1% sodium carbonate monohydrate for 35 seconds at 29.4° C. and the board is etched in ammoniacal etchant at pH greater than 9 for 2 minutes at 49° C.

## Claims

1. A compound comprising a photoinitiator joined to a hydrophilic carrier component.

2. The compound of claim 1, wherein the carrier component comprises a monomer, oligomer, polymer, plasticizer or surfactant.

3. The compound of claim 2, wherein the oligomer is a urethane oligomer.

4. The compound of claim 1, wherein the photoinitiator comprises an imidazole dimer, a benzophenone, an acetaphenone, an anthraquinone, a naphthaquinone, or a triazine-based compound.

5. The compound of claim 4, wherein the imidazole dimer is a hexaarylbiimidazole of the following formula: 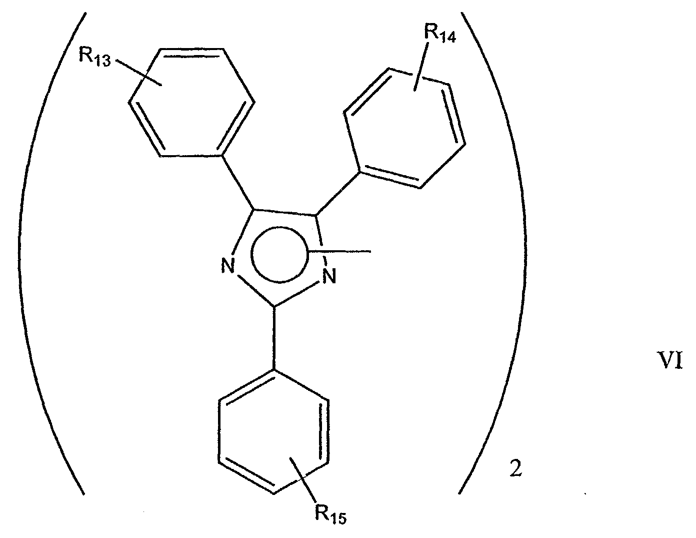 wherein R₁₃, R₁₄ and R₁₅ are the same or different and comprise hydrogen, unsubstituted or substituted alkyl, unsubstituted or substituted aryl, alkoxy, aryloxy, hydroxyl, aminyl, carboxyl, ester, thio, isocyanate, -N(CH₂―OCH₃)₂, hydroxyalkyloxy or alkylthio with the proviso that at least one of R₁₃, R₁₄ or R₁₅ is a reactive group that undergoes an addition or a condensation reaction with a reactive group of the carrier component to join the carrier component to the imidazole dimer, and R₁₅ also may be a halogen.

6. A photoresist comprising a compound with a photoinitiator joined to a hydrophilic carrier component.

7. The photoresist of claim 6, wherein the photoinitiator comprises an imidazole dimer, a benzophenone, an acetaphenone, an anthraquinone, naphthaquinone, a triazine-based compound, or mixtures thereof.

8. The photoresist of claim 7, wherein the photoinitiator is an imidazole dimer having the formula: 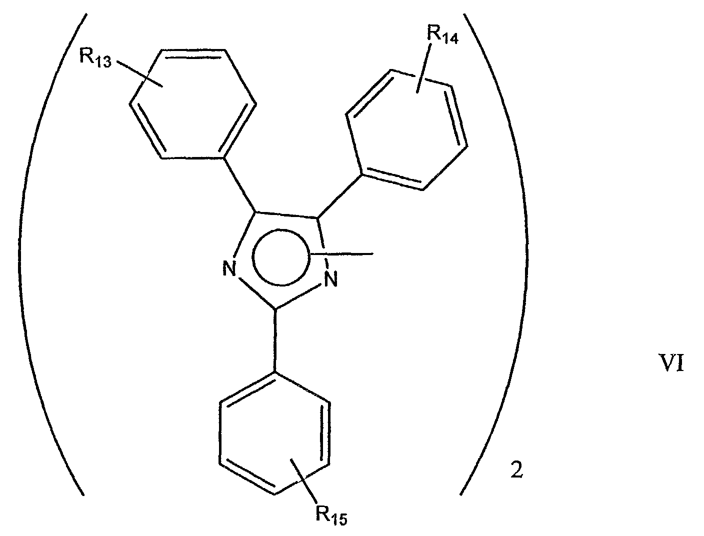 where R₁₃, R₁₄ and R₁₅ are the same or different and comprise hydrogen, unsubstituted or substituted alkyl, unsubstituted or substituted aryl, alkoxy, aryloxy, hydroxyl, aminyl, carboxyl, ester, thio, isocyanate, -N(CH₂―OCH₃)₂, hydroxyalkyloxy or alkylthio with the proviso that at least one of R₁₃, R₁₄ or R₁₅ is a reactive group which may undergo an addition or condensation reaction with a reactive group of the carrier component to join the imidazole dimer to the carrier component, and R₁₅ may also be a halogen.

9. A method of forming a pattern on a substrate comprising:
a) disposing on a substrate a photoresist composition with a hydrophilic carrier component bonded to a photoinitiator component;
b) imaging the photoresist; and
c) developing the photoresist

10. The method of claim 9, wherein the photoinitiator component comprises an imidazole dimer, a benzophenone, an acetophenone, an anthraquinone, a naphthaquinone, a triazine-based compound, or mixtures thereof.
